# EUROPEAN PATENT SPECIFICATION

(11) **EP 1 517 913 B1**
(45) Date of publication and mention of the grant of the patent: **21.02.2007**
(21) Application number: 00970619.3
(22) Date of filing: 06.10.2000
(51) Int. Cl.: C12N 15/63, C07K 19/00

(54) **A MYCOBACTERIUM TUBERCULOSIS CODING SEQUENCE FOR EXRESSION OF HETEROLOGOUS PROTEINS**
EINE MYCOBACTERIUM TUBERCULOSIS KODIERENDE SEQUENZ ZUR EXPRESSION VON HETEROLOGEN PROTEINEN
UNE SEQUENCE CODANTE DE MYCOBACTERIUM TUBERCULOSIS POUR FAVORISER UNE EXPRESSION DE PROTEINES HETEROLOGUES

(30) Priority: 07.10.1999 US 158585 P
(43) Date of publication of application: 30.03.2005
(73) Proprietor: CORIXA CORPORATION, Wilmington, DE 19808 (US)
(72) Inventor: SKEIKY, Yasir, Bellevue,WA 98006 (US); GUDERIAN, Jeffrey, Lynnwood, WA 98037 (US)
(74) Representative: Teuten, Andrew John
(86) International application number: PCT/US2000/027652
(87) International publication number: WO 2001/025401

(56) References cited:
- WO-A-01/24820
- WO-A-01/34802
- WO-A-01/51633
- WO-A-01/73032
- WO-A-01/90152
- WO-A-01/98460
- WO-A2-97/09429
- WO-A2-98/16645
- WO-A2-99/09186
- WO-A2-99/42076
- WO-A2-99/42118
- WO-A2-99/51748
- WANG A ET AL: "A novel method for increasing the expression level of recombinant proteins" PROTEIN EXPRESSION AND PURIFICATION, ACADEMIC PRESS, SAN DIEGO, CA, US, vol. 30, no. 1, July 2003 (2003-07), pages 124-133, XP004432813 ISSN: 1046-5928

## Description

### TECHNICAL FIELD

The present invention relates generally to nucleic acid and amino acid sequences of a fusion polypeptide comprising a *Mycobacterium tuberculosis* polypeptide, and a heterologous polypeptide of interest, expression vectors and host cells comprising such nucleic acids, and methods for producing such fusion polypeptides. In particular, the invention relates to materials and methods of using such *M. tuberculosis* sequence as a fusion partner to facilitate the stable and high yield expression of recombinant heterologous polypeptides of both eukaryotic and prokaryotic origin.

### BACKGROUND OF THE INVENTION

The advent of recombinant DNA technology has led to the molecular cloning of a large number of coding sequences or genes from diverse cell types. In order to study the function of these genes or to produce the products encoded by such sequences, these genes are inserted in expression vectors under the control of appropriate regulatory sequences. This transfer of the expression vector into a eukaryotic or prokaryotic host cell generally results in the expression of the encoded product which can be subsequently purified. Large-scale production of many gene products is particularly important in cases where such products are of medical or industrial value.

However, notwithstanding the advances in gene expression, certain coding sequences do not readily produce their products in stable form. For example, expression in *E coli* of recombinant proteins could be problematic particularly for proteins with trans-membrane domains or extensive hydrophobic sequences. Moreover, recombinant proteins may not contain the N-terminal amino acid residues with the appropriate codon bias. Thus, there remains a need for improved materials and methods for the expression of recombinant proteins.

WO99/42076 and WO99/51748 disclose the *M tuberculosis* antigen Ra12 and fusion proteins containing it.

### SUMMARY OF THE INVENTION

The present invention provides for the first time methods of using recombinant nucleic acid molecules that encode fusion polypeptides comprising a Ra12 polypeptide and a heterologous polypeptide, fusion polypeptides, expression vectors and host cells comprising the nucleic acid molecules, to produce stable and high yield expression of fusion polypeptides of interest.

In one aspect, the present invention provides a method for the stable and high yield expression of a recombinant heterologous polypeptide, the method comprising expressing in a host cell a recombinant nucleic acid molecule that encodes a fusion polypeptide, the fusion polypeptide comprising a Ra12 polypeptide and a heterologous polypeptide, wherein the Ra12 polypeptide is encoded by a Ra12 polynucleotide sequence that hybridizes to a sequence complementary to SEQ ID NO:3 under stringent conditions, and wherein the Ra12 polynucleotide sequence is located 5' to the heterologous polynucleotide sequence, which encodes the heterologous polypeptide. In another embodiment, the recombinant nucleic acid molecules further comprise a polynucleotide sequence that encodes a linker peptide between the Ra12 polynucleotide sequence and the heterologous polynucleotide sequence, wherein the linker peptide may comprise a cleavage site. In yet another embodiment, the recombinant nucleic acid molecules encode fusion polypeptides which further comprise an affinity tag. In yet another embodiment, the recombinant nucleic acid molecules encode a fusion polypeptide comprising a DPPD, a WT1, a mammaglobin, or a H9-32A heterologous polypeptide. In yet another embodiment, the recombinant nucleic acid molecules comprise a Ra12 polynucleotide sequence comprising at least about 30 nucleotides, at least about 60 nucleotides, or at least about 100 nucleotides. In yet another embodiment, the recombinant nucleic acid molecules comprise a Ra12 polynucleotide sequence as shown in SEQ ID NO:3. In yet another embodiment, the recombinant nucleic acid molecules comprise a Ra12 polynucleotide sequence that encodes a Ra12 polynucleotide as shown in SEQ ID NO:4, SEQ ID NO:17 or SEQ ID NO:18.

In a preferred embodiment, the host cell is *E. coli*.

In one embodiment, the Ra12 polypeptide comprises at least about 10 amino acids, at least about 30 amino acids, or at least about 100 amino acids. In another embodiment, the Ra12 polypeptide has a sequence as shown in SEQ ID NO:4, SEQ ID NO:17, or SEQ ID NO:18.

In one embodiment, the method further comprises purifying fusion polypeptides after their expression. In another embodiment, the method further comprises cleaving a fusion polypeptide between a Ra12 polypeptide and a heterologous polypeptide.

These and other aspects of the present invention will become apparent upon reference to the following detailed description and attached drawings.

### BRIEF DESCRIPTION OF THE DRAWINGS

Figure 1 illustrates a nucleotide sequence (SEQ ID NO:1) and an amino acid sequence (SEQ ID NO:2) of MTB32A.
Figure 2 illustrates a nucleotide sequence (SEQ ID NO:3) and an amino acid sequence (SEQ ID NO:4) of Ra12.
Figure 3 illustrates a recombinant nucleic acid sequence comprising a nucleotide sequence (SEQ ID NO:5) and an amino acid sequence (SEQ ID NO:6) of Ra12-DPPD fusion polypeptide.
Figure 4 illustrates a recombinant nucleic acid sequence comprising a nucleotide sequence (SEQ ID NO:7) and an amino acid sequence (SEQ ID NO:8) of Ra12-WT1 fusion polypeptide.
Figure 5 illustrates a recombinant nucleic acid sequence comprising a nucleotide sequence (SEQ ID NO:9) and an amino acid sequence (SEQ ID NO:10) of Ra12-mammaglobin fusion polypeptide.
Figure 6 illustrates a recombinant nucleic acid sequence comprising a nucleotide sequence (SEQ ID NO:11) and an amino acid sequence (SEQ ID NO:12) of Ra12-H9-32A fusion polypeptide.
Figure 7 illustrates Ra12(short) polypeptide (SEQ ID NO:17), which has amino acids 1-30 of SEQ ID NO:3.
Figures 8 illustrates Ra12(long) polypeptide (SEQ ID NO:18), which has amino acids 1-128 of SEQ ID NO:4.
Figure 9 illustrates a construct of Ra12 (short) polynucleotide fused to a human mammaglobin gene.

### DETAILED DESCRIPTION OF THE INVENTION

As noted above, the present invention provides for the first time methods for using Ra12 sequences as a fusion partner to facilitate the stable and high yield expression of recombinant heterologous polypeptides of both eukaryotic and prokaryotic origin.

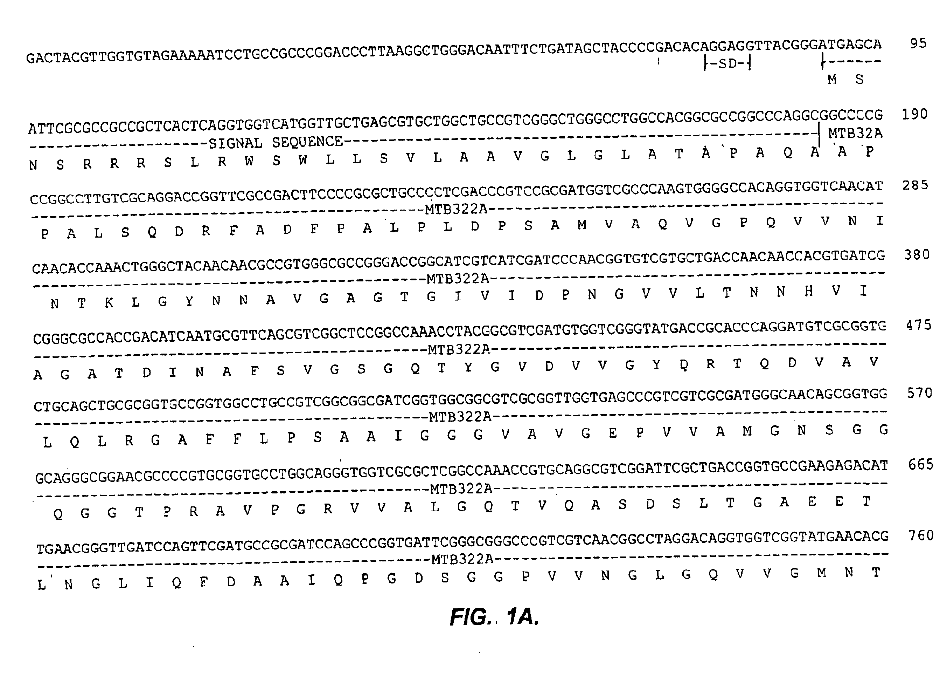

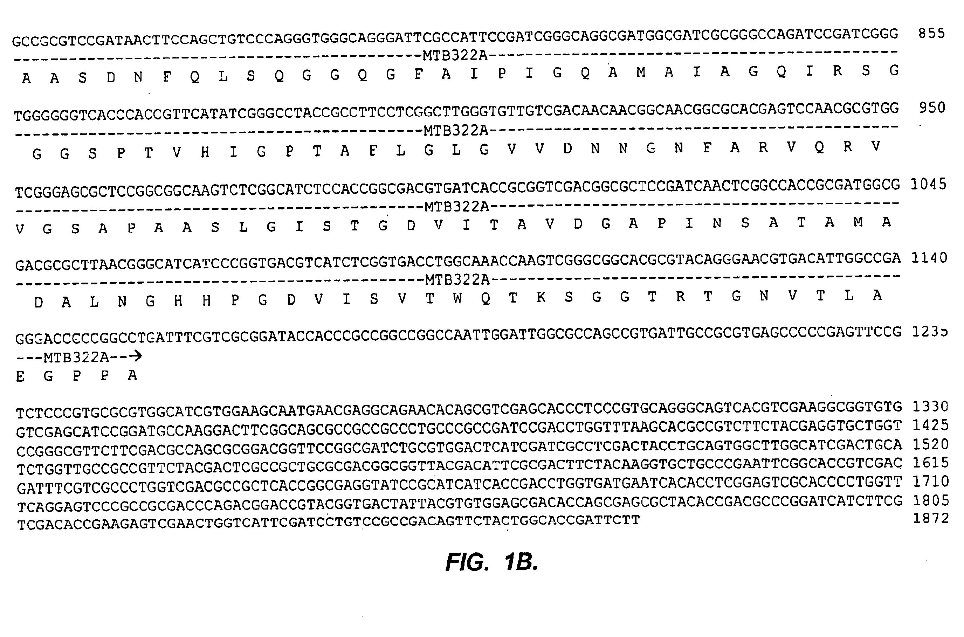

MTB32A is a serine protease of 32 KD molecular weight encoded by a gene in virulent and avirulent strains of *M tuberculosis.* The complete nucleotide sequence (SEQ ID NO:1) and amino acid sequence (SEQ ID NO:2) of MTB32A are disclosed in Figure 1. *See, also*, Skeiky *et al, Infection and Immun*. (1999) 67:3998-4007, This protein is naturally secreted into the supernatant of bacterial cultures. The open reading frame of the coding sequence contains N-terminal hydrophobic secretory signals. It stimulates peripheral blood mononuclear cells from healthy purified protein derivative (PPD)-positive donors to proliferate and secrete interferon. Thus, MTB32A is a candidate antigen for use in vaccine development against tuberculosis.

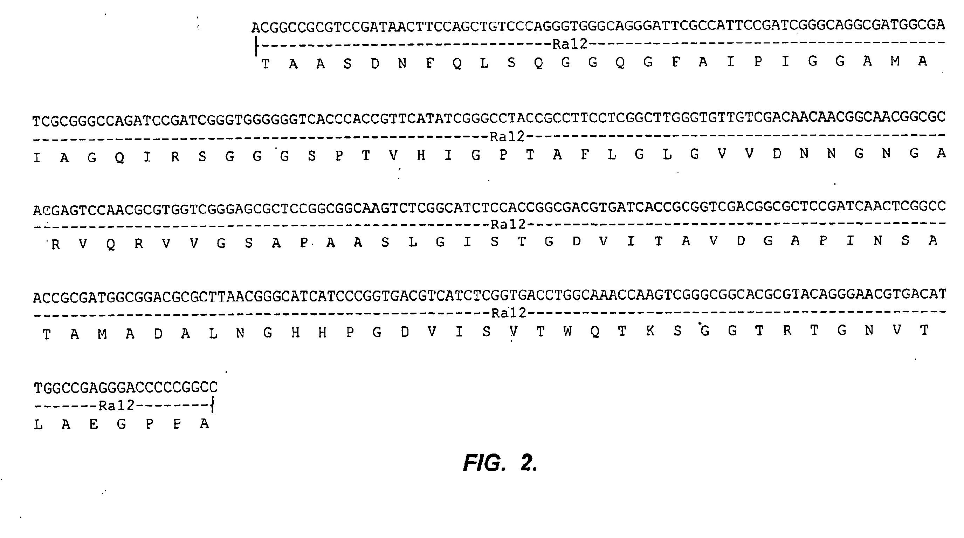

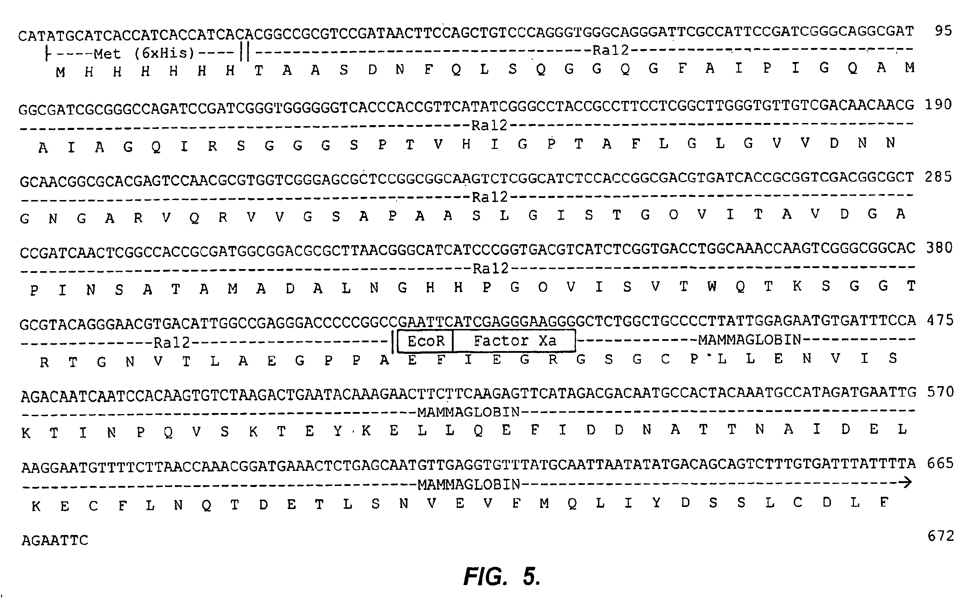

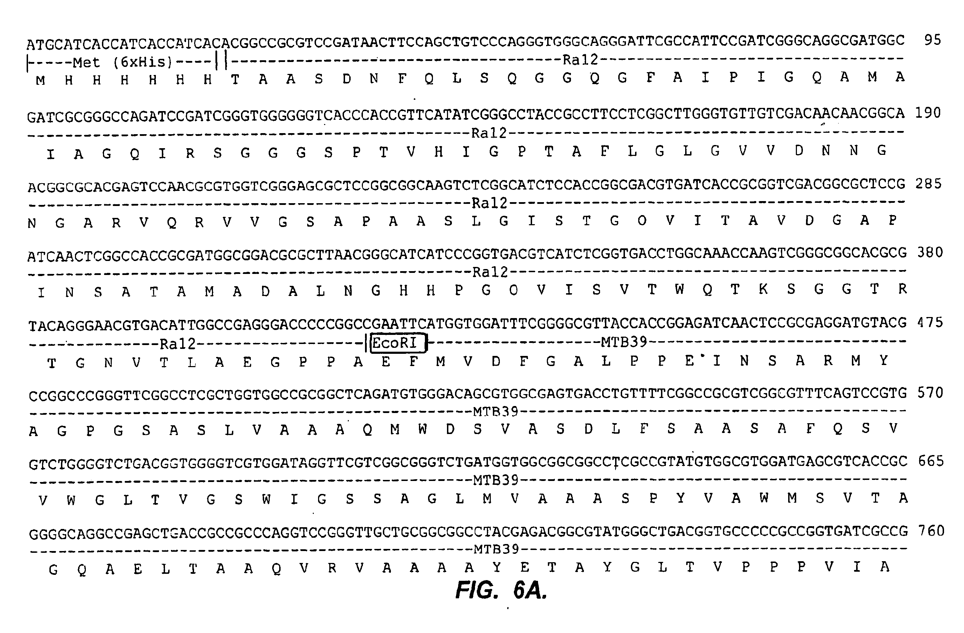

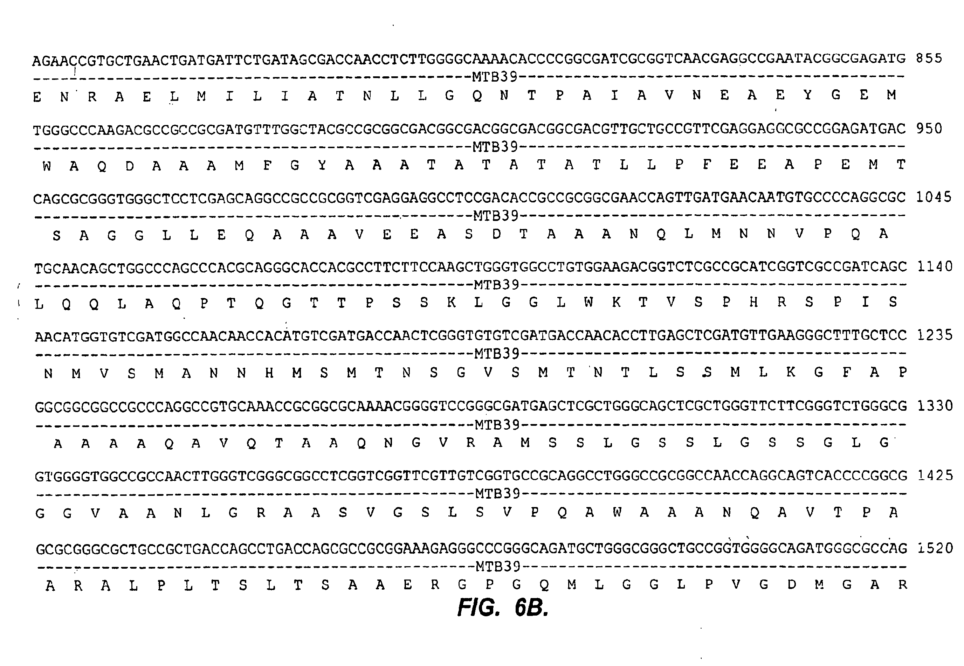

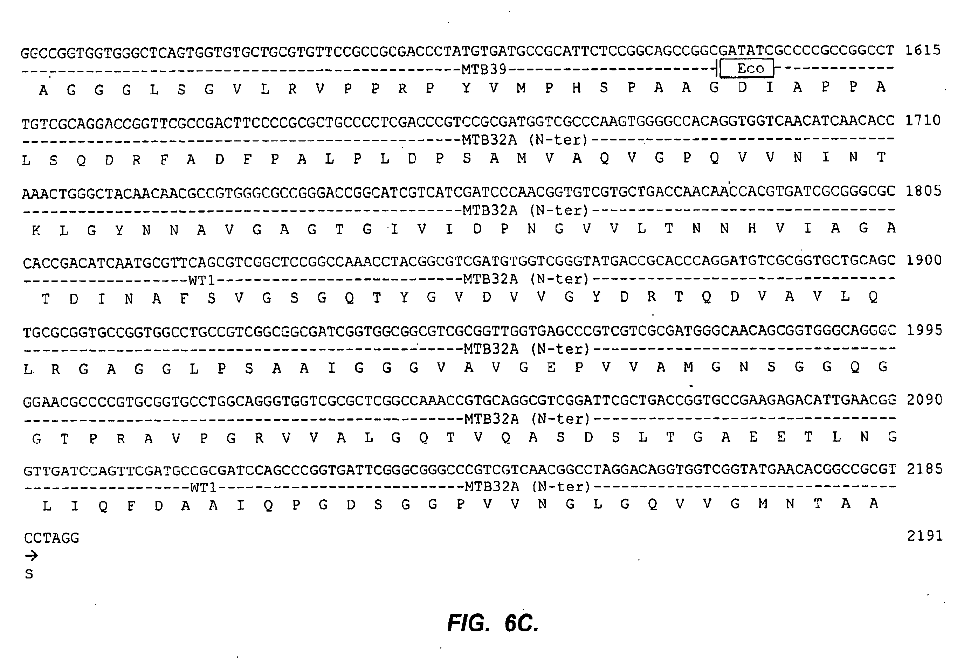

Surprisingly, it was discovered by the present inventors that a 14 KD C-terminal fragment of the MTB32A coding sequence expresses at high levels on its own and remains as a soluble protein throughout the purification process. This 14 KD C-terminal fragment of the MTB32A is referred herein as Ra12 (having amino acid residues 192 to 323 of MTB32A). The nucleic acid and amino acid sequences of native Ra12 are shown, *e*.*g*., in Figures 2-6. As described in detail below, the term "Ra12 polypeptide" or "Ra12 polynucleotide" as used herein refer to the native Ra12 sequences *(e.g.,* SEQ ID NO:3 or SEQ ID NO:4), their variants, or fragments thereof *(e.g.,* SEQ ID NO:17 or SEQ ID NO: 18). The present invention utilizes these properties of Ra12 polypeptides and provides methods for stable and high yield expression of fusion polypeptides comprising a Ra12 polypeptide and a heterologous polypeptide of interest. The methods of the present invention are particularly useful in expressing certain heterologous polypeptides (*e.g*., DPPD) that other conventional expression methods failed to express in any substantial quantity.

### Recombinant Fusion Nucleic Acids

Recombinant nucleic acids, which encode a fusion polypeptide comprising a Ra12 polypeptide and a heterologous polypeptide of interest, can be readily constructed by conventional genetic engineering techniques. Recombinant nucleic acids are constructed so that a Ra12 polynucleotide sequence is located 5' to a selected heterologous polynucleotide sequence.

In the present invention, any suitable heterologous polynucleotide of interest can be selected as a fusion partner to Ra12 nucleic acids to produce a fusion polypeptide. A "heterologous sequence" or a "heterologous nucleic acid," as used herein, is one that originates from a source foreign to the particular host cell, or, if from the same source, is modified from its original form. Thus, a heterologous nucleic acid in a prokaryotic host cell includes a heterologous nucleic acid that is endogenous to the particular host cell that has been modified. Modification of the heterologous sequence may occur, *e.g.,* by treating the DNA with a restriction enzyme to generate a DNA fragment that is capable of being operably linked to the promoter. Techniques such as site-directed mutagenesis are also useful in modifying a heterologous sequence.

A heterologous nucleic acid from both eukaryotic and prokaryotic origins can be selected as a fusion partner. These nucleic acids include, but are not limited to, nucleic acids that encode pathogenic antigens, bacterial antigens, viral antigens, cancer antigens, tumor antigens, and tumor suppressors. Exemplary heterologous nucleic acids of interest include DPPD, WT1, mammaglobin, H9-32A nucleic acids, and other *Mycobacterium tuberculosis* nucleic acids (*see, e.g.,* Cole *et al. Nature* (1999) 393:537-544; http://www.sanger.ac.uk; and http://www.pasteur.fr/mycdb/ for the complete genome sequences of *M. tuberculosis; see, also* WO98/53075 and WO98/53076, both of which are published on November 26, 1998 for nucleic acid sequences that encode *M. tuberculosis* proteins). Any one of the nucleic acids disclosed herein can be used alone or in combination as a heterologous nucleic acid that can be selected as a fusion partner.

In addition, any suitable Ra12 polynucleotide (*e.g*., native Ra12 polynucleotide having SEQ ID NO:3, variants or fragments thereof) can be used in constructing recombinant fusion nucleic acids of the present invention. Preferred Ra12 polynucleotides comprise at least about 15 consecutive nucleotides, at least about 30 nucleotides, at least about 60 nucleotides, at least about 100 nucleotides, at least about 200 nucleotides, or at least about 300 nucleotides. Polynucleotides may be single-stranded or double-stranded, and may be DNA (genomic, cDNA or synthetic) or RNA molecules.

In one embodiment, the Ra12 polynucleotide sequence is as shown in SEQ ID NO:3. In another embodiment, the Ra12 polynucleotide sequence encodes a Ra12 polypeptide as shown in SEQ ID NO:4. In some embodiments, the Ra12 polynucleotide sequence comprises a portion of SEQ ID NO:3 or encodes a portion of SEQ ID NO:4. For instance, a Ra12 polynucleotide comprising 90 nucleotides *(e.g.,* nucleotides 1-90 of SEQ ID NO:3), or a Ra12 polynucleotide comprising 384 nucleotides *(e.g.,* nucleotides 1-384 of SEQ ID NO:3) can be used as a fusion partner. *See* Examples 2 and 3 below.

Polynucleotides may comprise a native sequence (i.e., an endogenous sequence that encodes a Ra12 polypeptide SEQ ID NO:3 or a portion thereof) or may comprise a variant of such a sequence. Polynucleotide variants may contain one or more substitutions, additions, deletions and/or insertions such that the biological activity of the encoded fusion polypeptide is not diminished, relative to a fusion polypeptide comprising a native Ra12 polypeptide. Variants preferably exhibit at least about 70% identity, more preferably at least about 80% identity and most preferably at least about 90% identity to a polynucleotide sequence that encodes a native Ra12 polypeptide (SEQ ID NO:4) or a portion thereof. Optionally, the identity exists over a region that is at least about 25 to about 50 amino acids or nucleotides in length, or optionally over a region that is 75-100 amino acids or nucleotides in length.

Two polynucleotide or polypeptide sequences are said to be "identical" if the sequence of nucleotides or amino acids in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, eg 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 *Methods in Enzymology* vol. 183, Academic Press, Inc., San Diego, CA; Higgins, D.G. and Sharp, P.M. (1989) *CABIOS* 5:151-153; Myers, E.W. and Muller W. (1988) *CABIOS 4:11-17;* Robinson, E.D. (1971) *Comb. Theor* 11:105; Santou, N. Nes, M. (1987) *Mol. Biol. Evol.* 4:406-425; Sneath, P.H.A. and Sokal, R.R. (1973) *Numerical Taxonomy - the Principles and Practice of Numerical Taxonomy,* Freeman Press, San Francisco, CA; Wilbur, WJ. and Lipman, D.J. (1983) *Proc. Natl. Acad, Sci. USA 80:726-730*.

Alternatively, optimal alignment of sequences for comparison may be conducted by the local identity algorithm of Smith and Waterman (1981) *Add. APL. Math* 2:482, by the identity alignment algorithm of Needleman and Wunsch (1970) *J. Mol. Biol*. 48:443, by the search for similarity methods of Pearson and Lipman (1988) *Proc. Natl. Acad. Sci. USA* 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

Preferred examples of algorithms that are suitable for determining percent sequence identity and sequence similarity are the BLAST and BLAST 2.0 algorithms, which are described in Altschul *et al*. (1977) *Nucl. Acids Res.* 25:3389-3402 and Altschul *et al*. (1990) *J. Mol. Biol*. 215:403-410, respectively. BLAST and BLAST 2.0 can be used, for example with the parameters described herein, to determine percent sequence identity for the polynucleotides and polypeptides of the invention. Software for performing BLAST analyses is publicly available through the National Center for Biotechnology Information: For amino acid sequences, a scoring matrix can be used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment.

In one preferred approach, the "percentage of sequence identity" is determined by comparing two optimally aligned sequences over a window of comparison of at least 20 positions, wherein the portion of the polypeptide sequence in the comparison window may comprise additions or deletions (*i*.*e*., gaps) of 20 percent or less, usually 5 to 15 percent, or 10 to 12 percent, as compared to the reference sequences (which do not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the reference sequence *(i.e.,* the window size) and multiplying the results by 100 to yield the percentage of sequence identity.

Variants may also, or alternatively, be substantially homologous to a native Ra12 polynucleotide (*e*.*g*., SEQ ID NO:3), or a portion or complement thereof. Such polynucleotide variants are capable of hybridizing under stringent conditions to a naturally occurring DNA sequence encoding a native Ra12 polynucleotide (or a complementary sequence).

The phrase "selectively (or specifically) hybridizes to" refers to the binding, duplexing, or hybridizing of a molecule only to a particular nucleotide sequence under stringent hybridization conditions when that sequence is present in a complex mixture (*e*.*g*., total cellular or library DNA or RNA).

The phrase "stringent hybridization conditions" refers to conditions under which a probe will hybridize to its target subsequence, typically in a complex mixture of nucleic acid, but to no other sequences. Stringent conditions are sequence-dependent and will be different in different circumstances. Longer sequences hybridize specifically at higher temperatures. An extensive guide to the hybridization of nucleic acids is found in Tijssen, *Techniques in Biochemistry and Molecular Biology--Hybridization with Nucleic Probes*, "Overview of principles of hybridization and the strategy of nucleic acid assays" (1993). Generally, stringent conditions are selected to be about 5-10 °C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes *(e.g.,* 10 to 50 nucleotides) and at least about 60°C for long probes (*e.g*., greater than 50 nucleotides). Stringent conditions may also be achieved with the addition of destabilizing agents such as formamide. For selective or specific hybridization, a positive signal is at least two times background, preferably 10 times background hybridization. Exemplary stringent hybridization conditions can be as following: 50% formamide, 5x SSC, and 1% SDS, incubating at 42°C, or, 5x SSC, 1% SDS, incubating at 65°C, with a wash in 0.2x SSC, and 0.1% SDS at 65°C.

It will be appreciated by those of ordinary skill in the art that, as a result of the degeneracy of the genetic code, there are many nucleotide sequences that encode a Ra12 polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Thus, the terms such as "Ra12 polynucleotide" or "Ra12 polynucleotide sequence" as used herein refer to native Ra12 polynucleotide sequences (*e.g*., SEQ ID NO:3), fragments thereof, or any variants thereof. Functionally, any Ra12 polynucleotide has the ability to produce a fusion protein, and its ability to produce a fusion proteins in host cells may be enhanced or unchanged, relative to the native Ra12 polynucleotide (*e*.*g*., SEQ ID NO:3), or may be diminished by less than 50%, and preferably less than 20%, relative to the native Ra12 polynucleotide.

Nucleic acids encoding Ra12 polypeptides of this invention can be prepared by any suitable method known in the art. Exemplary methods include cloning and restriction of appropriate sequences or direct chemical synthesis by methods such as the phosphotriester method of Narang *et al*. (1979) *Meth. Enzymol*. 68: 90-99; the phosphodiester method of Brown *et al*. (1979) *Meth. Enzymol*. 68: 109-151; the diethylphosphoramidite method of Beaucage *et al*. (1981) *Tetra. Lett.,* 22: 1859-1862; and the solid support method of U.S. Patent No. 4,458,066.

In one embodiment, a nucleic acid encoding MTB32A or Ra12 is isolated by routine cloning methods. Nucleotide sequences of MTB32A or Ra12 as provided herein are used to provide probes that specifically hybridize to other MTB32A or Ra12 nucleic acids in a genomic DNA sample, or to a MTB32A mRNA or Ra12 mRNA in a total RNA sample (*e*.*g*., in a Southern or Northern blot). Once the target MTB32A or Ra12 nucleic acids are identified, it can be isolated according to standard methods known to those of skill in the art.

The desired nucleic acids can also be cloned using well known amplification techniques. Examples of protocols sufficient to direct persons of skill through *in vitro* amplification methods, including the polymerase chain reaction (PCR) the ligase chain reaction (LCR), Qβ-replicase amplification and other RNA polymerase mediated techniques are found in Berger, Sambrook, and Ausubel, as well as Mullis *et al.* (1987) U.S. Patent No. 4,683,202; *PCR Protocols A Guide to Methods and Applications* (Innis *et al.* eds) Academic Press Inc. San Diego, CA (1990) (Innis); Arnheim & Levinson (October 1, 1990) *C&EN* 36-47; *The Journal Of NIH Research* (1991) 3: 81-94; (Kwoh *et al.* (1989) *Proc. Natl. Acad. Sci. USA* 86: 1173; Guatelli *et al.* (1990) *Proc. Natl. Acad. Sci. USA* 87: 1874; Lomell *et al.* (1989) *J. Clin. Chem.* 35: 1826; Landegren *et al.* (1988) *Science* 241: 1077-1080; Van Brunt (1990) *Biotechnology* 8: 291-294; Wu and Wallace (1989) *Gene* 4: 560; and Barringer *et al.* (1990) *Gene* 89: 117. Improved methods of cloning *in vitro* amplified nucleic acids are described in Wallace *et al*., U.S. Pat. No. 5,426,039. Suitable primers for use in the amplification of the nucleic acids of the invention can be designed based on the sequences provided herein.

The MTB32A or Ra12 nucleic acids can also be cloned by detecting their expressed product by means of assays based on the physical, chemical, or immunological properties of the expressed protein. For example, one can identify a cloned MTB32A or Ra12 nucleic acid by the ability of a polypeptide encoded by the nucleic acid to bind with antisera or purified antibodies made against the MTB32A or Ra12 polypeptides provided herein, which also recognize and selectively bind to the MTB32A or Ra12 homologs.

In some embodiments, it may be desirable to modify the MTB32A or Ra12 nucleic acids of the invention. Altered nucleotide sequences which can be used in accordance with the invention include deletions, additions or substitutions of different nucleotide residues resulting in a sequence that encodes the same or a functionally equivalent gene product. The gene product itself may contain deletions, additions or substitutions of amino acid residues, which result in a silent change thus producing a functionally equivalent antigenic epitope. Such conservative amino acid substitutions may be made on the basis of similarity in polarity, charge, solubility, hydrophobicity, hydrophilicity, and/or the amphipathic nature of the residues involved. Preferably, Ra12 nucleic acids that are shorter in length than SEQ ID NO:3 and that encode biologically active fusion partner can be used. Such smaller functional equivalents of Ra12 polypeptides may be desirable to increase the amount of host cell resources that are available for the production of heterologous polypeptides of interest.

One of skill will recognize many ways of generating alterations in a given nucleic acid construct. Such well-known methods include site-directed mutagenesis, PCR amplification using degenerate oligonucleotides, exposure of cells containing the nucleic acid to mutagenic agents or radiation, chemical synthesis of a desired oligonucleotide *(e.g.,* in conjunction with ligation and/or cloning to generate large nucleic acids) and other well-known techniques. *See, e.g.,* Giliman and Smith (1979) *Gene* 8:81-97, Roberts *et al*. (1987) *Nature* 328: 731-734.

Recombinant nucleic acids that encode a fusion polypeptide comprising a Ra12 polypeptide and a selected heterologous polypeptide can be prepared using any methods known in the art. As described above, recombinant nucleic acids are constructed so that a Ra12 polynucleotide sequence is located 5' to a selected heterologous polynucleotide sequence. Ra12 and heterologous polynucleotide sequences can also be modified to facilitate their fusion and subsequent expression of fusion polypeptides. For example, the 3' stop codon of the Ra12 polynucleotide sequence can be substituted with an in frame linker sequence, which may provide restriction sites and/or cleavage sites. The recombinant nucleic acids can further comprise other nucleotide sequences such as sequences that encode affinity tags to facilitate protein purification protocols.

### Expression Vectors and Host Cells

The recombinant nucleic acids as described herein can be joined to a variety of other nucleotide sequences using established recombinant DNA techniques. For example, a polynucleotide can be cloned into any of a variety of cloning vectors, including plasmids, phagemids, lambda phage derivatives and cosmids. Vectors of particular interest include expression vectors, replication vectors, probe generation vectors and sequencing vectors. In general, a vector will contain an origin of replication functional in at least one organism, convenient restriction endonuclease sites and one or more selectable markers. Other elements will depend on the desired use, and will be apparent to those of ordinary skill in the art.

DNA sequences encoding the polypeptide components may be assembled separately, and ligated into an appropriate expression vector. The 3' end of the DNA sequence encoding one polypeptide component is ligated, with or without a polynucleotide sequence encoding a peptide linker, to the 5' end of a DNA sequence encoding the second polypeptide component so that the reading frames of the sequences are in phase. This permits translation into a single fusion protein that retains the biological activity of both component polypeptides.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

Depending on the host/vector system utilized, any of a number of suitable transcription and translation elements, including constitutive and inducible promoters, may be used in the expression vector. For example, when cloning in bacterial systems, inducible promoters such as pL of bacteriophage λ, plac, ptrp, ptac (ptrp-lac hybrid promoter; cytomegalovirus promoter) and the like may be used; when cloning in yeast cell systems, promoters such as ADHI, PGK, PHO5, or the α factor promoter may be used; when cloning in insect cell systems, promoters such as the baculovirus polyhedron promoter may be used; when cloning in plant cell systems, promoters derived from the genome of plant cells (*e*.*g*., heat shock promoters; the promoter for the small subunit of RUBISCO; the promoter for the chlorophyll α/β binding protein) or from plant viruses (*e.g*., the 35S RNA promoter of CaMV; the coat protein promoter of TMV) may be used; when cloning in mammalian cell systems, promoters derived from the genome of mammalian cells *(e.g.,* metallothionein promoter) or from mammalian viruses *(e.g.,* the adenovirus late promoter; the vaccinia virus 7.5K promoter) may be used; when generating cell lines that contain multiple copies of an antigen coding sequence, SV40-, BPV- and EBV-based vectors may be used with an appropriate selectable marker.

A variety of host-expression vector systems may be utilized to express Ra12 fusion protein coding sequences. These include, but are not limited to, microorganisms such as bacteria *(e.g., E. coli, B. subtilis)* transformed with recombinant bacteriophage DNA, plasmid DNA or cosmid DNA expression vectors containing a coding sequence; yeast *(e.g., Saccharomyces, Pichia)* transformed with recombinant yeast expression vectors containing a coding sequence; insect cell systems infected with recombinant virus expression vectors *(e.g.,* baculovirus) containing a coding sequence; plant cell systems infected with recombinant virus expression vectors *(e.g.,* cauliflower mosaic virus, CaMV; tobacco mosaic virus, TMV) or transformed with recombinant plasmid expression vectors *(e.g.,* Ti plasmid) containing a coding sequence; or mammalian cell systems *(e.g.,* COS, CHO, BHK, 293, 3T3 cells). The expression elements of these systems vary in their strength and specificities.

Bacterial systems are preferred for the expression of Ra12 fusion polypeptides. Commonly used prokaryotic control sequences, which are defined herein to include promoters for transcription initiation, optionally with an operator, along with ribosome binding site sequences, include such commonly used promoters as the beta-lactamase (penicillinase) and lactose *(lac)* promoter systems (Change *et al., Nature* (1977) 198: 1056), the tryptophan *(trp)* promoter system (Goeddel *et al., Nucleic Acids Res.* (1980) 8: 4057), the tac promoter (DeBoer *et al, Proc. Natl. Acad Sci. U.S.A.* (1983) 80:21-25); and the lambda-derived P_{L} promoter and N-gene ribosome binding site (Shimatake *et al., Nature* (1981) 292: 128). The particular promoter system is not critical to the invention; any available promoter that functions in prokaryotes can be used.

Either constitutive or regulated promoters can be used in the present invention. Regulated promoters can be advantageous because the host cells can be grown to high densities before expression of the Ra12 fusion polypeptides is induced. High level expression of heterologous proteins slows cell growth in some situations. Regulated promoters especially suitable for use in *E. coli* include the bacteriophage lambda P_{L} promoter, the hybrid *trp-lac* promoter (Amann *et al*., Gene (1983) 25: 167; de Boer *et al*., *Proc. Natl Acad Sci. USA* (1983) 80: 21, and the bacteriophage T7 promoter (Studier *et al., J. Mol. Biol*. (1986); Tabor *et al*., (1985). These promoters and their use are discussed in Sambrook *et al*., (1989) *Molecular Cloning: A Laboratory Manual, 2nd Ed., Vols. 1-3,* Cold Spring Harbor Laboratory.

For expression of Ra12 fusion polypeptides in prokaryotic cells other than *E. coli,* a promoter that functions in the particular prokaryotic species is required. Such promoters can be obtained from genes that have been cloned from the species, or heterologous promoters can be used. For example, the hybrid *trp-lac* promoter functions in *Bacillus* in addition to *E. coli.*

A ribosome binding site (RBS) is conveniently included in the expression cassettes of the invention. An RBS in *E*. *coli,* for example, consists of a nucleotide sequence 3-9 nucleotides in length located 3-11 nucleotides upstream of the initiation codon (Shine and Dalgarno, *Nature* (1975) 254: 34; Steitz, *In Biological regulation and development: Gene expression* (ed. R.F. Goldberger), vol. 1, p. 349, 1979, Plenum Publishing, NY).

When large quantities of the Ra12 fusion protein are to be produced, vectors which direct the expression of high levels of fusion protein products that are readily purified may be desirable. Such vectors include, but are not limited to, the *E*. *coli* expression vector pUR278 (Ruther *et al*. (1983) *EMBO J.* 2:1791), in which a coding sequence may be ligated into the vector in frame with the *lacZ* coding region so that a hybrid protein is produced; pIN vectors (Inouye and Inouye (1985) *Nucleic Acids Res.* 13:3101-3109; Van Heeke and Schuster (1989) *J. Biol. Chem.* 264:5503-5509); and the like. pGEX vectors may also be used to express foreign polypeptides as fusion proteins with glutathione S-transferase (GST). In general, such fusion proteins are soluble and can be purified easily from lysed cells by adsorption to glutathione-agarose beads followed by elution in the presence of free glutathione. For certain applications, it may be desirable to cleave the heterologous polypeptide of interest from the Ra12 fusion polypeptide after purification. This can be accomplished by any of several methods known in the art. For example, the pGEX vectors are designed to include thrombin or factor Xa protease cleavage sites so that the cloned fusion polypeptide of interest can be released from the GST moiety. *See, e.g.,* Sambrook *et al., supra*.; Itakura *et al., Science* (1977) 198:1056; Goeddel *et al., Proc. Natl. Acad. Sci. USA* (1979) 76:106; Nagai *et al., Nature* (1984) 309:810; Sung *et al., Proc. Nati. Acad. Sci. USA* (1986) 83:561. Cleavage sites can be engineered into the recombinant nucleic acids for the fusion proteins at the desired point of cleavage.

### Fusion Polypeptides

Within the context of the present invention, a "fusion" polypeptide comprises at least two parts: a Ra12 polypeptide as described herein, and a heterologous polypeptide of interest. In a fusion polypeptide, a Ra12 polypeptide is preferably fused, directly or indirectly, to the amino terminus of a heterologous polypeptide of interest, although fusion to the carboxy terminus of the heterologous polypeptide or insertion of the heterologous polypeptide into a site within an Ra12 polypeptide may also be appropriate.

Any heterologous polypeptide of interest, either eukaryotic or prokaryotic origins, can be selected as a fusion partner to a Ra12 polypeptide. These heterologous polypeptides include, but are not limited to, pathogenic antigens, bacterial antigens, viral antigens, cancer antigens, tumor antigens, and tumor suppressors. Exemplary heterologous polypeptides include DPPD, WT1, mammaglobin, H9-32A polypeptides, or other *M. tuberculosis* proteins. Any one of these polypeptides can be used alone or in combination as a heterologous polypeptide that can be selected as a fusion partner.

As noted above, a fusion polypeptide may comprise a native Ra12 polypeptide *(e*.*g*., SEQ ID NO:4), a variant thereof, or a fragment thereof. A polypeptide "variant," as used herein, is a polypeptide that differs from a native Ra12 polypeptide in one or more substitutions, deletions, additions and/or insertions, such that the biological activity of the polypeptide is not substantially diminished. In other words, the ability of a variant to produce fusion polypeptide in host cells may be enhanced or unchanged, relative to the native Ra12 protein, or may be diminished by less than 50%, and preferably less than 20%, relative to the native Ra12 protein. Such variants may generally be identified by modifying one of the above polypeptide sequences and evaluating the level of fusion polypeptide production in host cells, such as in *E. coli.* Exemplary variants include those in which a small portion *(e*.*g*., 1-30 amino acids, preferably 5-15 amino acids) has been removed from the N- and/or C-terminal of the native Ra12 polypeptides. In one embodiment, variants of native Ra12 polypeptides comprise at least about 5 amino acids, at least about 10 amino acids, at least about 30 amino acids, at least about 50 amino acids, or at least about 100 amino acids.

In one embodiment, the Ra12 polypeptide sequence is as shown in SEQ ID NO:4. In another embodiment, the Ra12 polypeptide sequence comprises a portion of SEQ ID NO:4. For instance, an Ra12 polypeptide comprising 30 amino acids (*e.g*., amino acids 1-30 of SEQ ID NO:4) or an Ra12 polypeptide comprising 128 amino acids *(e.g.,* amino acids 1-128 of SEQ ID NO:4) can be used as a fusion partner. *See* Examples 2 and 3 below.

Polypeptide variants preferably exhibit at least about 70%, more preferably at least about 80% or at least about 90%, and most preferably at least about 95% identity (determined as described above) to the identified polypeptides. Optionally, identity exists over a region that is at least about 20 to about 50 amino acids in length, or optionally over a region that is 75-100 amino acids in length.

Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

Thus, the terms such as "Ra12 polypeptide" or "Ra12 polypeptide sequence" as used herein refer to native Ra12 polynucleotide sequences (*e*.*g*., SEQ ID NO:4), fragments thereof (e.g., SEQ ID NO:17 or 18), or any variants thereof. Functionally, a Ra12 polypeptide has the ability to produce a fusion protein, and its ability to produce a fusion protein in host cells may be enhanced or unchanged, relative to the native Ra12 polypeptide (*e.g*., SEQ ID NO:4), or may be diminished by less than 50%, and preferably less than 20%, relative to the native Ra12 polypeptide.

As noted above, fusion polypeptides may be conjugated to a linker or other sequence for ease of synthesis, purification or identification of the polypeptide or to enhance binding of the polypeptide to a solid support. For example, a peptide linker sequence may be employed to separate a Ra12 polypeptide and a heterologous polypeptide of interest by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. In certain embodiments, peptide linker sequences may contain Gly, Asn and Ser residues. Other near neutral amino acids, such as Thr and Ala may also be used in the linker sequence. Amino acid sequences which may be usefully employed as linkers include those disclosed in Maratea *et al., Gene 40*:39-46, 1985; Murphy *et al., Proc. Natl. Acad. Sci. USA 83*:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

In a preferred embodiment, a linker can provide a specific cleavage site between a Ra12 polypeptide and a heterologous polypeptide of interest. Such a cleavage site may contain a target for proteolytic enzyme that includes, for example, enterokinase, Factor Xa, trypsin, collagenase, thrombin, ubiquitin hydrolase; or for chemical cleavage agents such as, for example, cyanogen bromide or hydroxyamine.

A fusion polypeptide may optionally contain an affinity tag which is linked to the fusion polypeptide so that the purification of recombinant polypeptides can be simplified. For example, multiple histidine residues encoded by the tag allow the use of metal chelate affinity chromatography methods for the purification of fusion polypeptides. Other examples of affinity tag molecules include, Strep-tag, PinPoint, maltose binding protein, glutathione S-transferase, *etc. See, e.g.,* Glick and Pasternak (1999) *Molecular Biotechnology Principles and Applications of Recombinant DNA,* 2^{nd} Ed., American Society for Microbiology, Washington, DC.

Fusion polypeptides may be prepared using any of a variety of well known techniques. Recombinant fusion polypeptides encoded by DNA sequences as described above may be readily prepared from the DNA sequences using any of a variety of expression vectors known to those of ordinary skill in the art. Expression may be achieved in any appropriate host cell that has been transformed or transfected with an expression vector containing a DNA molecule that encodes a recombinant polypeptide. Suitable host cells include prokaryotes, yeast and higher eukaryotic cells described above. Preferably, the host cell employed is *E*. *coli.* Supernatants from suitable host/vector systems which secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

Portions and other variants having fewer than about 100 amino acids, and generally fewer than about 50 amino acids, may also be generated by synthetic means, using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain. *See* Merrifield, *J. Am. Chem. Soc. 85*:2149-2146, 1963. Equipment for automated synthesis of polypeptides is commercially available from suppliers such as Perkin Elmer/Applied BioSystems Division (Foster City, CA), and may be operated according to the manufacturer's instructions.

In general, polypeptides (including fusion proteins) and polynucleotides as described herein are isolated. An "isolated" polypeptide or polynucleotide is one that is removed from its original environment. For example, a naturally-occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

In addition to providing stable and high yield expression of fusion polypeptides of interest, the recombinant fusion nucleic acids and fusion polypeptides of the invention can be used in a number of other methods. For example, the fusion polypeptide coding sequence of the invention can be used to encode a protein product for use as an antigen for detecting serum antibodies. For example, the presence of serum antibodies to *M tuberculosis* antigens in an individual indicates that the individual is infected with *M tuberculosis.* In standard diagnostic tests, serum antibodies to *M. tuberculosis* are detected by monitoring binding of serum antibodies to *M tuberculosis* proteins. The fusion polypeptides of the invention are useful as sources of proteins for monitoring binding of serum antibodies to fusion proteins.

Alternatively, the fusion polypeptide can be used as an immunogen to induce and/or enhance immune responses. Such coding sequences can be ligated with a coding sequence of another molecule such as a *M*. *tuberculosis* antigen, a cytokine or an adjuvant. Such polynucleotides may be used *in vivo* as a DNA vaccine (U.S. Patent Nos. 5,589,466; 5,679,647; and 5,703,055). Alternatively, purified or partially purified fusion polypeptides or fragments may be used as vaccines or therapeutic compositions. Any of a variety of methods known in the art can be employed to produce vaccines or therapeutic compositions comprising the fusion polypeptides of the present invention.

### Protein Purification and Preparations

Once a recombinant protein is expressed, it can be identified by assays based on the physical or functional properties of the product, including radioactive labeling of the product followed by analysis by gel electrophoresis, radioimmunoassay, ELISA, bioassays, *etc.*

Once the encoded protein is identified, it may be isolated and purified by standard methods including chromatography (e.g., high performance liquid chromatography, ion exchange, affinity, and sizing column chromatography), centrifugation, differential solubility, or by any other standard technique for the purification of proteins. *See, generally*, R. Scopes, *Protein Purification*, Springer-Verlag, N.Y. (1982), Deutscher, *Methods in Enzymology Vol. 182: Guide to Protein Purification*, Academic Press, Inc. N.Y. (1990). The actual conditions used will depend, in part, on factors such as net charge, hydrophobicity, hydrophilicity, *etc.,* and will be apparent to those having skill in the art. The functional properties may be evaluated using any suitable assays.

The functional properties of the fusion protein may be evaluated using any suitable assay such as antibody binding, induction of T cell proliferation, stimulation of cytokine production such as IL2, IL-4 and IFN-γ. For the practice of the present invention, it is preferred that each fusion protein is at least 80% purified from other proteins. It is more preferred that they are at least 90% purified. For *in vivo* administration, it is preferred that the proteins are greater than 95% purified.

The purified proteins may be further processed before use. For example, the proteins may digested with a specific enzyme to separate the Ra12 polypeptide from the heterologous polypeptide.

One of skill would recognize that modifications can be made to the recombinant nucleic acids and fusion polypeptides without diminishing their biological activity. Some modifications may be made to facilitate the cloning, expression, or incorporation of the tag molecule into a fusion polypeptide. Such modifications are well known to those of skill in the art and include, for example, a methionine added at the amino terminus to provide an initiation site, or additional amino acids (*e.g*., poly His) placed on either terminus to create conveniently located restriction sites or termination codons or purification sequences.

The following Examples are offered by way of illustration and not by way of limitation.

### EXAMPLES

The following examples describe experiments that illustrate that Ra12 fusion constructs produced stable and high yield expression of fusion polypeptides. The following examples also illustrate that various Ra12 sequences can be used as a fusion partner.

### EXAMPLE 1: The Full Length Ra12 Sequence (SEQ ID NO:4) as a Fusion Partner A. Construction of Expression Vectors

Coding sequences of *M. tuberculosis* antigens were modified by PCR in order to facilitate their fusion and subsequent expression of fusion protein. pET 17b vector (Novagen) was modified to include Ra12, a 14 kDa C-terminal fragment of the serine protease antigen MTB32A of *M. tuberculosis.* The 3' stop codon of the Ra12 sequence was substituted with an in frame EcoRI site and the N-terminal end was engineered to code for six His-tag residues immediately following the initiator Met to facilitate a simple one step purification protocol of Ra12 recombinant proteins by affinity chromatography over Ni-NTA matrix.

Specifically, the C-terminal fragment of antigen MTB32A was amplified by standard PCR methods using the oligonucleotide primers 5' CAA TTA CAT ATG **CAT CAC CAT CAC CAT CAC** ACG GCC GCG TCC GAT AAC TTC (SEQ ID NO:13) and the 3' oligonucleotide sequence is 5'-CTA ATC GAA TTC GGC CGG GGG TCC CTC GGC CAA (SEQ ID NO:14). The 450 bp product was digested with NdeI and EcoRI and cloned into the pET 17b expression vector similarly digested with the same enzymes. Expression of the recombinant Ra12 protein was accomplished following transformation into the *E. coli* BL-21 (pLysE) host cells (Novagen) and induction with IPTG. Following lysis of the *E. coli* cells and centrifugation at 10K rpm, recombinant Ra12 was found in the soluble supernatant fraction. Protein from the soluble supernatant was purified by affinity chromatography over an Ni-NTA column which remained soluble following dialysis in 1 x PBS. The amount of purified protein obtained was routinely in the 60 to 100 mg per liter range.

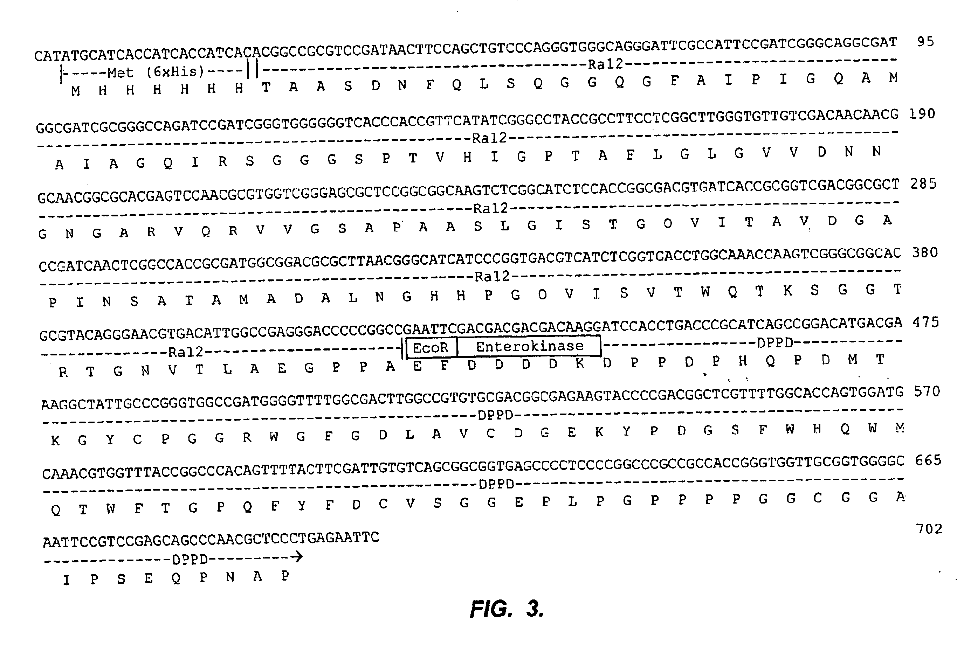

DPPD sequence was engineered for expression as a fusion protein with Ra12 by designing oligonucleotide primers to specifically amplify the mature secreted form. The 5' oligonucleotide containing an enterokinase recognition site (DDDK) has the sequences 5'-CAA TTA GAA TTC GAC GAC GAC GAC AAG GAT CCA CCT GAC CCG CAT CAG-3' (SEQ ID NO:15) and the 3' oligonucleotide sequence is 5'CAA TTA GAA TTC TCA GGG AGC GTT GGG CTG CTC (SEQ ID NO:16). The resulting PCR amplified product was digested with EcoRI and subcloned into the EcoRI site of the pET-Ra12 vector. Following transformation into the *E. coli* host strain (XL1-blue; Stratagene), clones containing the correct size insert were submitted for sequencing in order to identify those that were in frame with the Ra12 fusion. Subsequently, the DNA of interest (Fig. 3) was transformed into the BL-21 (pLysE) bacterial host and fusion protein expressed following induction of the culture with IPTG.

### B. Expression and Purification of Fusion Proteins

The recombinant (His-tag) Ra12-DPPD fusion protein was purified from 500 ml of IPTG induced batch cultures from the soluble supernatant by affinity chromatography using the one step QIAexpress Ni-NTA Agarose matrix (QIAGEN, Chatsworth, CA) in the presence of 8M urea. Briefly, 20 ml of an overnight saturated culture of BL21 containing the pET construct was added into 500 ml of 2xYT media containing 50 ug/ml ampicillin and 34 ug/ml chloramphenicol, grown at 37°C with shaking. The bacterial cultures were induced with 2mM IPTG at an OD 560 of 0.3 and grown for an additional 3 h (OD 1.3 to 1.9). Cells were harvested from 500 ml batch cultures by centrifugation and resuspended in 20 ml of binding buffer (0.1 M sodium phosphate, pH 8.0; 10 mM Tris-HCl, pH 8.0) containing 2mM PMSF and 20 ug/ml leupeptin. *E. coli* was lysed by adding 15 mg of lysozyme and rocking for 30 min at 4°C following sonnication (4 x 30 sec). Lysed cells were spun at 12 k rpm for 30 min and urea was added directly to the supernatant at a final concentration of 8M.

The supernatant was batch bound to Ni-NTA agarose resin (5 ml resin per 500 ml inductions) by rocking at R/T for 1 h and the matrix passed over a column. The flow through was passed twice over the same column followed by three washes with 30 ml each of wash buffer (0.1 M sodium phosphate and 10 mM Tris-HCL, pH 6.3) also containing 8 M urea. Bound protein was eluted with 30 ml of 100 mM imidazole in wash buffer and 5 ml fractions collected. Fractions containing the recombinant antigen were pooled, dialyzed against 10 mM Tris-HCl (pH 8.0) bound one more time to the Ni-NTA matrix, eluted and dialyzed in 1xPBS (pH 7.4) or 10 mM Tris-HCL (pH 7.8). The yield of the purified recombinant fusion protein was in the 50 to 75 mg per liter of induced bacterial culture with greater than 95% purity representing a single band. Recombinant proteins were assayed for endotoxin contamination using the Limulus assay (BioWhittaker) and were shown to contain < 10 E.U./mg (< 1 ng LPS/mg).

### C. Generation of Antiserum

The purified fusion protein (100 ug) was mixed with 100 ug of muramyl dipeptide, brought up to 1 ml with 1 x PBS and emulsified with 1 ml IFA (incomplete freunds; Life Technologies) adjuvant. The emulsion was injected at multiple sites s.c. into a female New Zealand rabbit (R&R Rabbitry, Stanwood, WA). The rabbit was given two subsequent boosters (100 ug antigen in IFA) 6 weeks apart and a final i.v. shot with 100 ug of the recombinant protein again given after 6 weeks. One week after the final boost, the rabbit was sacrificed and serum was collected and stored at -20°C.

### D. Immunoblotting Analysis

*M tuberculosis* (strain H37Rv) total lysate or PPD (2.5 µg each) and 25 ng of the purified recombinant Ra12-DPPD fusion protein were separated by electrophoresis on 16% SDS-PAGE gels and transferred to nitrocellulose using a semi-dry transfer apparatus (BioRad). Blots, in duplicate, were blocked for a minimum of 1 hr with PBS/0.1% Tween and probed with polyclonal sera from the same rabbit prior to immunization or post immunization with the purified recombinant fusion protein (diluted 1:500 in PBS/0. 1% Tween 20). Reactivity was assessed as previously using [¹²⁵I]-protein A, followed by autoradiography.

### E. Results

Several expression systems were initially evaluated for the expression of DPPD in *E*. *coli.* This included sub-cloning of DPPD coding sequence as non-fusion constructs in 1) pET 17b (Novagen) and pQ30 (Qiagen, Santa Clarita, CA) or 2) as fusion constructs using pET32A (Novagen, Madison, WI) or pGEX-2T (Pharmacia Biotech, Piscataway, NJ). In all of these systems, very little if any DPPD was expressed and purified.

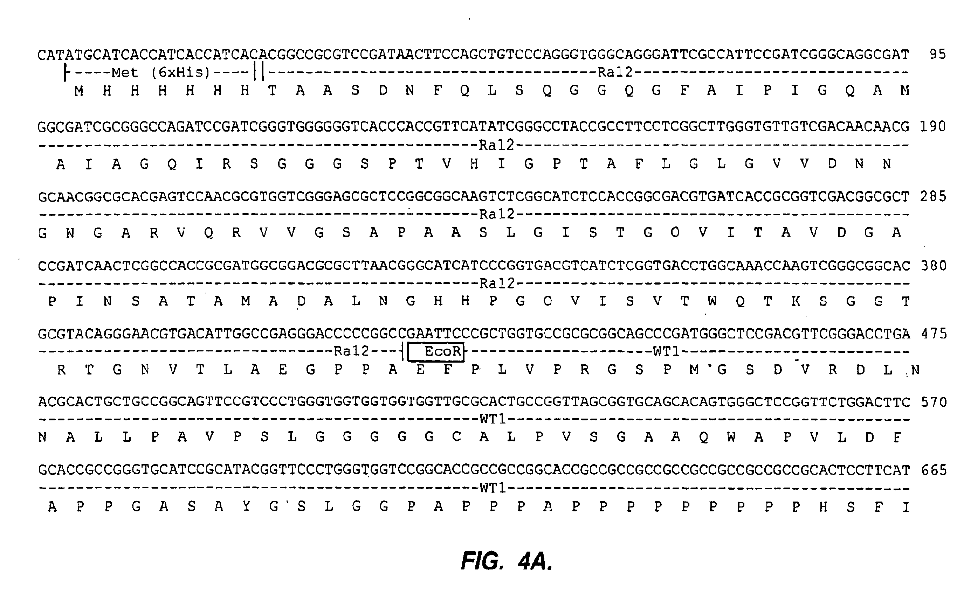

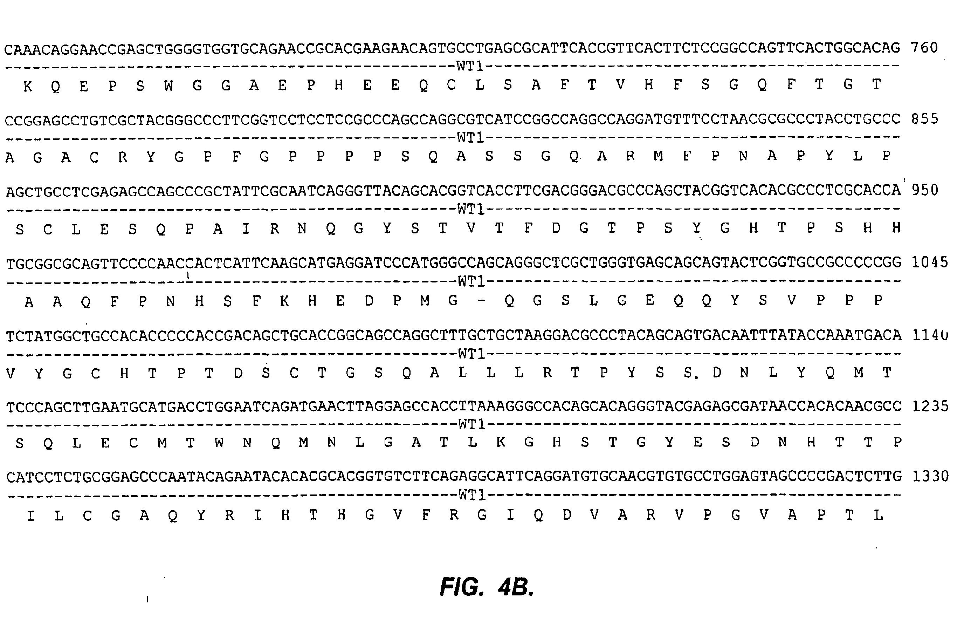

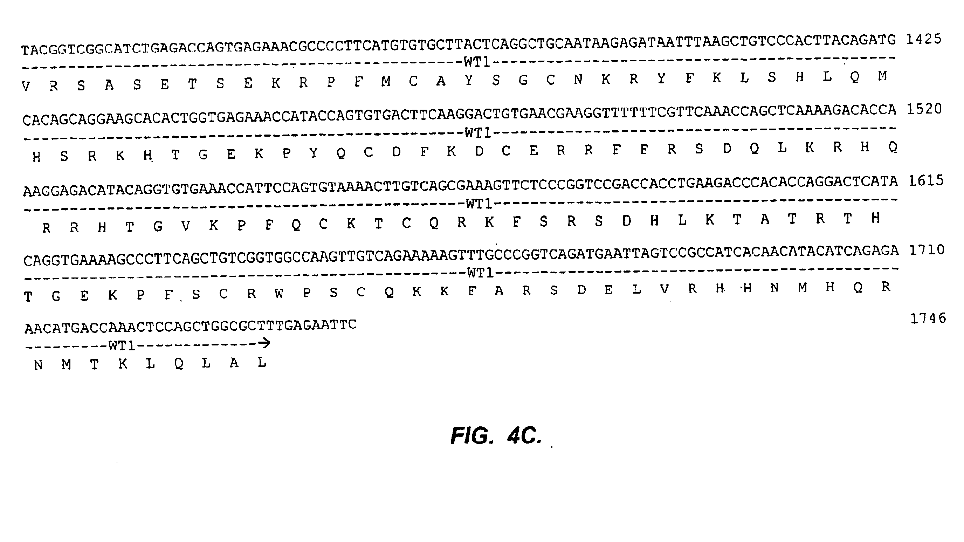

In contrast, when the DPPD coding sequence was inserted 3' to the Ra12 sequence in an expression vector and transformed into *E. coli*, a large amount of Ra12-DPPD fusion protein was produced. The nucleotide sequence (SEQ ID NO:5) and amino acid sequence (SEQ ID NO:6) of Ra12-DPPD are disclosed in Figure 3. The immunogenicity of DPPD was maintained as evidenced by the ability of antiserum to react with the purified protein in immunoblotting analysis. In addition, three other proteins of eukaryotic or prokaryotic origin (*see* Figures 4-6) were also successfully expressed by the Ra12 fusion constructs. Thus, the Ra12 coding sequence is useful as a fusion partner in an expression construct to facilitate the expression of a heterologous sequence.

### EXAMPLE 2: Short Ra12 Polypeptide (SEQ ID NO:17) as a Fusion Partner

In this example, a Ra12 polypeptide comprising amino acids 1-30 of SEQ ID NO:4 was used as a fusion partner to link with the full length human mammaglobin gene. This short form of Ra12 polypeptide has the amino acid sequence shown in SEQ ID NO:17, and is referred to herein as "Ra12(short)".

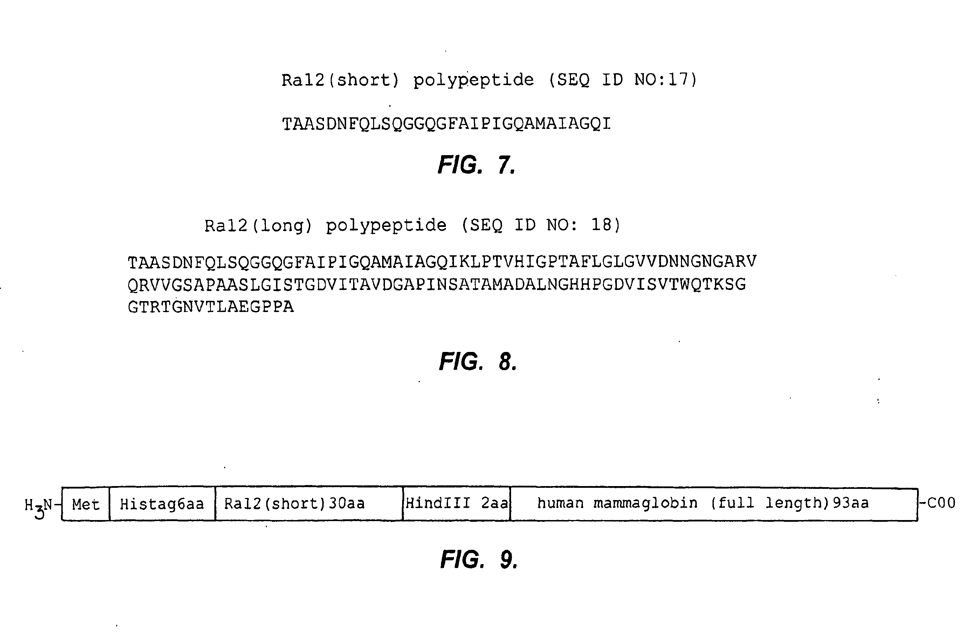

As shown in Figure 9, the 3' end of the Ra12(short) sequence is fused to the full length human mammaglobin gene. Specifically, the human mammaglobin gene was amplified by standard PCR methods using the following oligonucleotide primers: the 5' primer, Hind III site: 5'-gcgaagcttATGAAGTTGCTGATGGTCCTCATGC-3' (SEQ ID NO:19); the 3' primer, XhoI site: 5'-cggctcgagTTAAAATAAATCACAAAGACTGCTGTC-3' (SEQ ID NO:20). The 5' Hind III and 3' Xho I sites were added to assist subcloning into a vector. The N-terminal end of the fusion construct was engineered to code for six His-tag residues immediately following the Met to facilitate purification protocols. The expression of the fusion construct was accomplished following transformation into E. coli using procedures similar to those described in Example 1. Compared to a construct without a Ra12(short) sequence, the fusion construct with a Ra12(short) sequence substantially increased the expression of the fusion Ra12(short)-mammaglobin protein.

### EXAMPLE 3: Longer Ra12 Polypeptide (SEQ ID NO: 18) as a Fusion Partner

In this example, a Ra12 polypeptide comprising amino acids 1-128 of SEQ ID NO:4 was used as a fusion partner to link with the full length human mamma globin gene. This long form of Ra12 polypeptide has the ammo acid sequence shown in SEQ ID NO:18, and is referred to herein as "Ra12(long)". Cloning and expression procedures similar to those described in Example 2 were used. Compared to a construct without a Ra12(long) sequence, the fusion construct with a Ra12(long) sequence substantially increased the expression of the Ra12(long)-mammaglobin fusion protein.

The present invention is not to be limited in scope by the exemplified embodiments which are intended as illustrations of aspects of the invention, and any clones, nucleotide or amino acid sequences which are functionally equivalent can also be used. Indeed, various modifications of the invention in addition to those described herein will become apparent to those skilled in the art from the foregoing description and accompanying drawings. It is also to be understood that all base pair sizes given for nucleotides are approximate and are used for purposes of description.

## Claims

1. A method for the stable and high yield expression of a recombinant heterologous polypeptide, the method comprising expressing in a host cell a recombinant nucleic acid molecule that encodes a fusion polypeptide, the fusion polypeptide comprising a Ra12 polypeptide and a heterologous polypeptide, wherein the Ra12 polypeptide is encoded by a Ra12 polynucleotide sequence that hybridizes to a sequence complementary to SEQ ID NO:3 under stringent conditions, and wherein the Ra12 polynucleotide sequence is located 5' to the heterologous polynucleotide sequence which encodes the heterologous polypeptide.

2. The method according to claim 1, wherein the fusion polypeptide further comprises an affinity tag which is linked to the fusion polypeptide.

3. The method according to claim 1 or claim 2, wherein the fusion polypeptide is purified from the host cell.

4. The method according to any one of claims 1 to 3, wherein the host cell is *E. coli.*

5. The method according to any one of claims 1 to 4, wherein the recombinant nucleic acid molecule further comprises a polynucleotide sequence that encodes a linker peptide between the Ra12 polynucleotide sequence and the heterologous polynucleotide sequence.

6. The method according to claim 5, wherein the linker peptide comprises a cleavage site.

7. The method according to claim 1, wherein the heterologous nucleic acid sequence encodes a DPPD, a WT1, a mammaglobin, or a H9-32A polypeptide.

8. The method according to any one of claims 1 to 7, wherein the Ra12 polynucleotide sequence comprises at least 30 nucleotides.

9. The method according to claim 8, wherein the Ra12 polynucleotide sequence comprises at least 60 nucleotides.

10. The method according to claim 8, wherein the Ra12 polynucleotide sequence comprises at least 100 nucleotides.

11. The method according to any one of claims 1 to 10, wherein the Ra12 polynucleotide sequence encodes a Ra12 polypeptide as shown in SEQ ID NO: 17.

12. The method according to any one of claims 1 to 10, wherein the Ra12 polynucleotide sequence encodes a Ra12 polypeptide as shown in SEQ ID NO: 18.

13. The method according to any one of claims 1 to 10, wherein the Ra12 polynucleotide sequence is as shown in SEQ ID NO:3.

14. The method according to any one of claims 1 to 10, wherein the Ra12 polynucleotide sequence encodes a Ra12 polypeptide as shown in SEQ ID NO:4 or a variant having at least 90% amino acid sequence identity thereto.

15. The method according to claim 14, wherein the Ra12 polynucleotide sequence encodes a Ra12 polypeptide as shown in SEQ ID NO:4 or a variant having at least 95% amino acid sequence identity thereto.

16. The method according to any one of claims 1 to 7, wherein the Ra12 polypeptide comprises at least 10 amino acids.

17. The method according to claim 16, wherein the Ra12 polypeptide comprises at least 30 amino acids.

18. The method according to claim 16, wherein the Ra12 polypeptide comprises at least 100 amino acids.

19. The method according to any one of claims 1 to 7, wherein the Ra12 polypeptide has a sequence as shown in SEQ ID NO:4.

20. The method according to any one of claims 1 to 7, wherein the Ra12 polypeptide has a sequence as shown in SEQ ID NO: 17.

21. The method according to any one of claims 1 to 7, wherein the Ra12 polypeptide has a sequence as shown in SEQ ID NO: 18.

22. The method according to any one of claims 1 to 21, the method further comprising cleaving the fusion polypeptide between the Ra12 polypeptide and the heterologous polypeptide.

23. Use of a Ra12 polynucleotide sequence that hybridizes to a sequence complementary to SEQ ID NO:3 under stringent conditions in a method for the stable and high yield expression of a recombinant heterologous polypeptide, the method comprising expressing in a host cell a recombinant nucleic acid molecule that encodes a fusion polypeptide, the fusion polypeptide comprising a Ra12 polypeptide encoded by said Ra12 polynucleotide and a heterologous polypeptide, wherein said Ra12 polynucleotide sequence is located 5' to the heterologous polynucleotide sequence which encodes the heterologous polypeptide.

## Patentansprüche

1. Verfahren zur stabilen Expression mit hoher Ausbeute eines rekombinanten heterologen Polypeptids, wobei man in dem Verfahren ein für ein Fusionspolypeptid codierendes rekombinantes Nukleinsäuremolekül in einer Wirtszelle exprimiert, wobei das Fusionspolypeptid ein Ra12-Polypeptid und ein heterologes Polypeptid umfaßt, wobei das Ra12-Polypeptid von einer Ra12-Polynukleotidsequenz codiert wird, die unter stringenten Bedingungen an eine zu SEQ ID NO:3 komplementäre Sequenz hybridisiert, und wobei die Ra12-Polynukleotidsequenz 5' zu der für das heterologe Polypeptid codierenden heterologen Polynukleotidsequenz lokalisiert ist.

2. Verfahren nach Anspruch 1, wobei das Fusionspolypeptid ferner ein Affinitäts-Tag umfaßt, das mit dem Fusionspolypeptid verknüpft ist.

3. Verfahren nach Anspruch 1 oder Anspruch 2, wobei das Fusionspolypeptid aus der Wirtszelle aufgereinigt wird.

4. Verfahren nach einem der Ansprüche 1 bis 3, wobei es sich bei der Wirtszelle um *E. coli* handelt.

5. Verfahren nach einem der Ansprüche 1 bis 4, wobei das rekombinante Nukleinsäuremolekül ferner eine Polynukleotidsequenz umfaßt, die für ein Linkerpeptid zwischen der Ra12-Polynukleotidsequenz und der heterologen Polynukleotidsequenz codiert.

6. Verfahren nach Anspruch 5, wobei das Linkerpeptid eine Spaltstelle enthält.

7. Verfahren nach Anspruch 1, wobei die heterologe Nukleinsäuresequenz für ein DPPD-, ein WT1-, ein Mammaglobin- oder ein H9-32A-Polypeptid codiert.

8. Verfahren nach einem der Ansprüche 1 bis 7, wobei die Ra12-Polynukleotidsequenz wenigstens 30 Nukleotide umfaßt.

9. Verfahren nach Anspruch 8, wobei die Ra12-Polynukleotidsequenz wenigstens 60 Nukleotide umfaßt.

10. Verfahren nach Anspruch 8, wobei die Ra12-Polynukleotidsequenz wenigstens 100 Nukleotide umfaßt.

11. Verfahren nach einem der Ansprüche 1 bis 10, wobei die Ra12-Polynukleotidsequenz für ein wie in SEQ ID NO: 17 dargestelltes Ra12-Polypeptid codiert.

12. Verfahren nach einem der Ansprüche 1 bis 10, wobei die Ra12-Polynukleotidsequenz für ein wie in SEQ ID NO: 18 dargestelltes Ra12-Polypeptid codiert.

13. Verfahren nach einem der Ansprüche 1 bis 10, wobei es sich bei der Ra12-Polynukleotidsequenz um die wie in SEQ ID NO: 3 dargestellte Sequenz handelt.

14. Verfahren nach einem der Ansprüche 1 bis 10, wobei die Ra12-Polynukleotidsequenz für ein wie in SEQ ID NO: 4 dargestelltes Ra12-Polypeptid oder eine Variante mit wenigstens 90% Aminosäuresequenzidentität dazu codiert.

15. Verfahren nach Anspruch 14, wobei die Ra12-Polynukleotidsequenz für ein wie in SEQ ID NO: 4 dargestelltes Ra12-Polypeptid oder eine Variante mit wenigstens 95% Aminosäuresequenzidentität dazu codiert.

16. Verfahren nach einem der Ansprüche 1 bis 7, wobei das Ra12-Polypeptid wenigstens 10 Aminosäuren umfaßt.

17. Verfahren nach Anspruch 16, wobei das Ra12-Polypeptid wenigstens 30 Aminosäuren umfaßt.

18. Verfahren nach Anspruch 16, wobei das Ra12-Polypeptid wenigstens 100 Aminosäuren umfaßt.

19. Verfahren nach einem der Ansprüche 1 bis 7, wobei das Ra12-Polypeptid eine wie in SEQ ID NO: 4 dargestellte Sequenz aufweist.

20. Verfahren nach einem der Ansprüche 1 bis 7, wobei das Ra12-Polypeptid eine wie in SEQ ID NO: 17 dargestellte Sequenz aufweist.

21. Verfahren nach einem der Ansprüche 1 bis 7, wobei das Ra12-Polypeptid eine wie in SEQ ID NO: 18 dargestellte Sequenz aufweist.

22. Verfahren nach einem der Ansprüche 1 bis 21, bei dem man ferner das Fusionspolypeptid zwischen dem Ra12-Polypeptid und dem heterologen Polypeptid spaltet.

23. Verwendung einer Ra12-Polynukleotidsequenz, die unter stringenten Bedingungen an eine zu SEQ ID NO: 3 komplementäre Sequenz hybridisiert, in einem Verfahren zur stabilen Expression mit hoher Ausbeute eines rekombinanten heterologen Polypeptids, wobei man in dem Verfahren ein für ein Fusionspolypeptid codierendes rekombinantes Nukleinsäuremolekül in einer Wirtszelle exprimiert, wobei das Fusionspolypeptid ein von der Ra12-Polynukleotidsequenz codiertes Ra12-Polypeptid und ein heterologes Polypeptid umfaßt, wobei die Ra12-Polynukleotidsequenz 5' zu der für das heterologe Polypeptid codierenden heterologen Polynukleotidsequenz lokalisiert ist.

## Revendications

1. Procédé pour l'expression stable et à rendement élevé d'un polypeptide recombinant hétérologue, le procédé comprenant l'expression dans une cellule hôte d'une molécule d'acide nucléique recombinante qui code pour un polypeptide de fusion, le polypeptide de fusion comprenant un polypeptide Ra12 et un polypeptide hétérologue, **caractérisé en ce que** le polypeptide Ra12 est codé par une séquence polynucléotidique Ra12 qui s'hybride avec une séquence complémentaire à SEQ ID NO : 3 dans des conditions stringentes, et **caractérisé en ce que** la séquence polynucléotidique Ra12 est située en 5' de la séquence polynucléotidique hétérologue qui code pour le polypeptide hétérologue.

2. Procédé selon la revendication 1, **caractérisé en ce que** le polypeptide de fusion comprend en outre une étiquette d'affinité qui est liée au polypeptide de fusion.

3. Procédé selon la revendication 1 ou la revendication 2, **caractérisé en ce que** le polypeptide de fusion est purifié à partir de la cellule hôte.

4. Procédé selon l'une quelconque des revendications 1 à 3, **caractérisé en ce que** la cellule hôte est *E. coli.*

5. Procédé selon l'une quelconque des revendications 1 à 4, **caractérisé en ce que** la molécule d'acide nucléique recombinante comprend en outre une séquence polynucléotidique qui code pour un peptide de liaison entre la séquence polynucléotidique Ra12 et la séquence polynucléotidique hétérologue.

6. Procédé selon la revendication 5, **caractérisé en ce que** le peptide de liaison comprend un site de clivage.

7. Procédé selon la revendication 1, **caractérisé en ce que** la séquence d'acide nucléique hétérologue code pour un polypeptide DPPD, WT1, mammaglobine ou H9-32A.

8. Procédé selon l'une quelconque des revendications 1 à 7, **caractérisé en ce que** la séquence polynucléotidique Ra12 comprend au moins 30 nucléotides.

9. Procédé selon la revendication 8, **caractérisé en ce que** la séquence polynucléotidique Ra12 comprend au moins 60 nucléotides.

10. Procédé selon la revendication 8, **caractérisé en ce que** la séquence polynucléotidique Ra12 comprend au moins 100 nucléotides.

11. Procédé selon l'une quelconque des revendications 1 à 10, **caractérisé en ce que** la séquence polynucléotidique Ra12 code pour un polypeptide Ra12 tel que représenté dans SEQ ID NO : 17.

12. Procédé selon l'une quelconque des revendications 1 à 10, **caractérisé en ce que** la séquence polynucléotidique Ra12 code pour un polypeptide Ra12 tel que représenté dans SEQ ID NO : 18.

13. Procédé selon l'une quelconque des revendications 1 à 10, **caractérisé en ce que** la séquence polynucléotidique Ra12 est telle que représentée dans SEQ ID NO : 3.

14. Procédé selon l'une quelconque des revendications 1 à 10, **caractérisé en ce que** la séquence polynucléotidique Ra12 code pour un polypeptide Ra12 tel que représenté dans SEQ ID NO : 4 ou un variant ayant une identité de séquence d'acides aminés d'au moins 90 % avec celui-ci.

15. Procédé selon la revendication 14, **caractérisé en ce que** la séquence polynucléotidique Ra12 code pour un polypeptide Ra12 tel que représenté dans SEQ ID NO : 4 ou un variant ayant une identité de séquence d'acides aminés d'au moins 95 % avec celui-ci.

16. Procédé selon l'une quelconque des revendications 1 à 7, **caractérisé en ce que** le polypeptide Ra12 comprend au moins 10 acides aminés.

17. Procédé selon la revendication 16, **caractérisé en ce que** le polypeptide Ra12 comprend au moins 30 acides aminés.

18. Procédé selon la revendication 16, **caractérisé en ce que** le polypeptide Ra12 comprend au moins 100 acides aminés.

19. Procédé selon l'une quelconque des revendications 1 à 7, **caractérisé en ce que** le polypeptide Ra12 possède une séquence telle que représentée dans SEQ ID NO : 4.

20. Procédé selon l'une quelconque des revendications 1 à 7, **caractérisé en ce que** le polypeptide Ra12 possède une séquence telle que représentée dans SEQ ID NO : 17.

21. Procédé selon l'une quelconque des revendications 1 à 7, **caractérisé en ce que** le polypeptide Ra12 possède une séquence telle que représentée dans SEQ ID NO : 18.

22. Procédé selon l'une quelconque des revendications 1 à 21, le procédé comprenant en outre le clivage du polypeptide de fusion entre le polypeptide Ra12 et le polypeptide hétérologue.

23. Utilisation d'une séquence polynucléotidique Ra12 qui s'hybride avec une séquence complémentaire à SEQ ID NO : 3 dans des conditions stringentes, dans un procédé pour l'expression stable et à rendement élevé d'un polypeptide recombinant hétérologue, le procédé comprenant l'expression dans une cellule hôte d'une molécule d'acide nucléique recombinante qui code pour un polypeptide de fusion, le polypeptide de fusion comprenant un polypeptide Ra12 codé par ladite séquence polynucléotidique Ra12 et un polypeptide hétérologue, **caractérisée en ce que** ladite séquence polynucléotidique Ra12 est située en 5' de la séquence polynucléotidique hétérologue qui code pour le polypeptide hétérologue.


### SEQUENCE LISTING

<110> Corixa Corporation
<120> Methods of Using a Mycobacterium Tuberculosis Coding Sequence to Facilitate Stable and High Yield Expression of Heterologous Proteins
<130> AHB/FP6050272
<140> EP 00970619.3
   <141> 2000-10-06
<150> PCT/US00/27652
   <151> 2060-10-06
<150> US 60/158,585
   <151> 1999-10-07
<160> 22
<170> PatentIn Ver. 2.1
<210> 1
   <211> 1872
   <212> DNA
   <213> Mycobacterium tuberculosis
<220>
   <223> 32 KD serine protease MTB32A
<220>
   <221> CDS
   <222> (89)..(1156)
   <223> MTB32A
<220>
   <221> sig_peptide
   <222> (89)..(184)
   <223> N-terminal hydrophobic secretory signal sequence
<220>
   <221> mat_peptide
   <222> (185)..(1153)
<400> 1
   gactacgttg gtgtagaaaa atcctgccgc ccggaccctt aaggctggga caatttctga 60
   tagctacccc gacacaggag gttacggg atg agc aat tcg cgc cgc cgc tca     112
                                  Met Ser Asn Ser Arg Arg Arg Ser
                                          -30                 -25
   ctc agg tgg tca tgg ttg ctg agc gtg ctg gct gcc gtc ggg ctg ggc     160
   Leu Arg Trp Ser Trp Leu Leu Ser Val Leu Ala Ala Val Gly Leu Gly
               -20                 -15                 -10
   ctg gcc acg gcg ccg gcc cag gcg gcc ccg ccg gcc ttg tcg cag gac     208
   Leu Ala Thr Ala Pro Ala Gln Ala Ala Pro Pro Ala Leu Ser Gln Asp
           -5                  -1   1               5
   cgg ttc gcc gac ttc ccc gcg ctg ccc ctc gac ccg tcc gcg atg gtc     256
   Arg Phe Ala Asp Phe Pro Ala Leu Pro Leu Asp Pro Ser Ala Met Val
   10 15 20
   gcc caa gtg ggg cca cag gtg gtc aac atc aac acc aaa ctg ggc tac 304
   Ala Gln Val Gly Pro Gln Val Val Asn Ile Asn Thr Lys Leu Gly Tyr
   25 30 35 40
   aac aac gcc gtg ggc gcc ggg acc ggc atc gtc atc gat ccc aac ggt 352
   Asn Asn Ala Val Gly Ala Gly Thr Gly Ile Val Ile Asp Pro Asn Gly
   45 50 55
   gtc gtg ctg acc aac aac cac gtg atc gcg ggc gcc acc gac atc aat 400
   Val Val Leu Thr Asn Asn His Val Ile Ala Gly Ala Thr Asp Ile Asn
   60 65 70
   gcg ttc agc gtc ggc tcc ggc caa acc tac ggc gtc gat gtg gtc ggg 448
   Ala Phe Ser Val Gly Ser Gly Gln Thr Tyr Gly Val Asp Val Val Gly
   75 80 85
   tat gac cgc acc cag gat gtc gcg gtg ctg cag ctg cgc ggt gcc ggt 496
   Tyr Asp Arg Thr Gln Asp Val Ala Val Leu Gln Leu Arg Gly Ala Gly
   90 95 100
   ggc ctg ccg tcg gcg gcg atc ggt ggc ggc gtc gcg gtt ggt gag ccc 544
   Gly Leu Pro Ser Ala Ala Ile Gly Gly Gly Val Ala Val Gly Glu Pro
   105 110 115 120
   gtc gtc gcg atg ggc aac agc ggt ggg cag ggc gga acg ccc cgt gcg 592
   Val Val Ala Met Gly Asn Ser Gly Gly Gln Gly Gly Thr Pro Arg Ala
   125 130 135
   gtg cct ggc agg gtg gtc gcg ctc ggc caa acc gtg cag gcg tcg gat 640
   Val Pro Gly Arg Val Val Ala Leu Gly Gln Thr Val Gln Ala Ser Asp
   140 145 150
   tcg ctg acc ggt gcc gaa gag aca ttg aac ggg ttg atc cag ttc gat 688
   Ser Leu Thr Gly Ala Glu Glu Thr Leu Asn Gly Leu Ile Gln Phe Asp
   155 160 165
   gcc gcg atc cag ccc ggt gat tcg ggc ggg ccc gtc gtc aac ggc cta 736
   Ala Ala Ile Gln Pro Gly Asp Ser Gly Gly Pro Val Val Asn Gly Leu
   170 175 180
   gga cag gtg gtc ggt atg aac acg gcc gcg tcc gat aac ttc cag ctg 784
   Gly Gln Val Val Gly Met Asn Thr Ala Ala Ser Asp Asn Phe Gln Leu
   185 190 195 200
   tcc cag ggt ggg cag gga ttc gcc att ccg atc ggg cag gcg atg gcg 832
   Ser Gln Gly Gly Gln Gly Phe Ala Ile Pro Ile Gly Gln Ala Met Ala
   205 210 215
   atc gcg ggc cag atc cga tcg ggt ggg ggg tca ccc acc gtt cat atc 880
   Ile Ala Gly Gln Ile Arg Ser Gly Gly Gly Ser Pro Thr Val His Ile
   220 225 230
   ggg cct acc gcc ttc ctc ggc ttg ggt gtt gtc gac aac aac ggc aac 928
   Gly Pro Thr Ala Phe Leu Gly Leu Gly Val Val Asp Asn Asn Gly Asn
   235 240 245
   ggc gca cga gtc caa cgc gtg gtc ggg agc gct ccg gcg gca agt ctc 976
   Gly Ala Arg Val Gln Arg Val Val Gly Ser Ala Pro Ala Ala Ser Leu
   250 255 260
   ggc atc tcc acc ggc gac gtg atc acc gcg gtc gac ggc gct ccg atc    1024
   Gly Ile Ser Thr Gly Asp Val Ile Thr Ala Val Asp Gly Ala Pro Ile
   265                 270                 275                 280
   aac tcg gcc acc gcg atg gcg gac gcg ctt aac ggg cat cat ccc ggt    1072
   Asn Ser Ala Thr Ala Met Ala Asp Ala Leu Asn Gly His His Pro Gly
                   285                 290                 295
   gac gtc atc tcg gtg acc tgg caa acc aag tcg ggc ggc acg cgt aca    1120
   Asp Val Ile Ser Val Thr Trp Gln Thr Lys Ser Gly Gly Thr Arg Thr
               300                 305                 310
   ggg aac gtg aca ttg gcc gag gga ccc ccg gcc tga tttcgtcgcg         1166
   Gly Asn Val Thr Leu Ala Glu Gly Pro Pro Ala
           315                 320
   gataccaccc gccggccggc caattggatt ggcgccagcc gtgattgccg cgtgagcccc 1226
   cgagttccgt ctcccgtgcg cgtggcatcg tggaagcaat gaacgaggca gaacacagcg 1286
   tcgagcaccc tcccgtgcag ggcagtcacg tcgaaggcgg tgtggtcgag catccggatg 1346
   ccaaggactt cggcagcgcc gccgccctgc ccgccgatcc gacctggttt aagcacgccg 1406
   tcttctacga ggtgctggtc cgggcgttct tcgacgccag cgcggacggt tccggcgatc 1466
   tgcgtggact catcgatcgc ctcgactacc tgcagtggct tggcatcgac tgcatctggt 1526
   tgccgccgtt ctacgactcg ccgctgcgcg acggcggtta cgacattcgc gacttctaca 1586
   aggtgctgcc cgaattcggc accgtcgacg atttcgtcgc cctggtcgac gccgctcacc 1646
   ggcgaggtat ccgcatcatc accgacctgg tgatgaatca cacctcggag tcgcacccct 1706
   ggtttcagga gtcccgccgc gacccagacg gaccgtacgg tgactattac gtgtggagcg 1766
   acaccagcga gcgctacacc gacgcccgga tcatcttcgt cgacaccgaa gagtcgaact 1826
   ggtcattcga tcctgtccgc cgacagttct actggcaccg attctt                1872
<210> 2
   <211> 355
   <212> PRT
   <213> Mycobacterium tuberculosis
   <223> 32 KD serine protease MTB32A
<400> 2
   Met Ser Asn Ser Arg Arg Arg Ser Leu Arg Trp Ser Trp Leu Leu Ser
   1 5 10 15
   Val Leu Ala Ala Val Gly Leu Gly Leu Ala Thr Ala Pro Ala Gln Ala
   20 25 30
   Ala Pro Pro Ala Leu Ser Gln Asp Arg Phe Ala Asp Phe Pro Ala Leu
   35 40 45
   Pro Leu Asp Pro Ser Ala Met Val Ala Gln Val Gly Pro Gln Val Val
   50 55 60
   Asn Ile Asn Thr Lys Leu Gly Tyr Asn Asn Ala Val Gly Ala Gly Thr
   65 70 75 80
   Gly Ile Val Ile Asp Pro Asn Gly Val Val Leu Thr Asn Asn His Val
   85 90 95
   Ile Ala Gly Ala Thr Asp Ile Asn Ala Phe Ser Val Gly Ser Gly Gln
   100 105 110
   Thr Tyr Gly Val Asp Val Val Gly Tyr Asp Arg Thr Gln Asp Val Ala
   115 120 125
   Val Leu Gln Leu Arg Gly Ala Gly Gly Leu Pro Ser Ala Ala Ile Gly
   130 135 140
   Gly Gly Val Ala Val Gly Glu Pro Val Val Ala Met Gly Asn Ser Gly
   145 150 155 160
   Gly Gln Gly Gly Thr Pro Arg Ala Val Pro Gly Arg Val Val Ala Leu
   165 170 175
   Gly Gln Thr Val Gln Ala Ser Asp Ser Leu Thr Gly Ala Glu Glu Thr
   180 185 190
   Leu Asn Gly Leu Ile Gln Phe Asp Ala Ala Ile Gln Pro Gly Asp Ser
   195 200 205
   Gly Gly Pro Val Val Asn Gly Leu Gly Gln Val Val Gly Met Asn Thr
   210 215 220
   Ala Ala Ser Asp Asn Phe Gln Leu Ser Gln Gly Gly Gln Gly Phe Ala
   225 230 235 240
   Ile Pro Ile Gly Gln Ala Met Ala Ile Ala Gly Gln Ile Arg Ser Gly
   245 250 255
   Gly Gly Ser Pro Thr Val His Ile Gly Pro Thr Ala Phe Leu Gly Leu
   260 265 270
   Gly Val Val Asp Asn Asn Gly Asn Gly Ala Arg Val Gln Arg Val Val
   275 280 285
   Gly Ser Ala Pro Ala Ala Ser Leu Gly Ile Ser Thr Gly Asp Val Ile
   290 295 300
   Thr Ala Val Asp Gly Ala Pro Ile Asn Ser Ala Thr Ala Met Ala Asp
   305 310 315 320
   Ala Leu Asn Gly His His Pro Gly Asp Val Ile Ser Val Thr Trp Gln
   325 330 335
   Thr Lys Ser Gly Gly Thr Arg Thr Gly Asn Val Thr Leu Ala Glu Gly
   340 345 350
   Pro Pro Ala
   355
<210> 3
   <211> 396
   <212> DNA
   <213> Mycobacterium tuberculosis
<220>
   <223> 14 KD C-terminal fragment of MTB32A Ra12
<220>
   <221> CDS
   <222> (1)..(396)
   <223> Ra12
<400> 3
   acg gcc gcg tcc gat aac ttc cag ctg tcc cag ggt ggg cag gga ttc      48
   Thr Ala Ala Ser Asp Asn Phe Gln Leu Ser Gln Gly Gly Gln Gly Phe
   1               5                   10                  15
   gcc att ccg atc ggg cag gcg atg gcg atc gcg ggc cag atc cga tcg      96
   Ala Ile Pro Ile Gly Gln Ala Met Ala Ile Ala Gly Gln Ile Arg Ser
               20                  25                  30
   ggt ggg ggg tca ccc acc gtt cat atc ggg cct acc gcc ttc ctc ggc     144
   Gly Gly Gly Ser Pro Thr Val His Ile Gly Pro Thr Ala Phe Leu Gly
           35                  40                  45
   ttg ggt gtt gtc gac aac aac ggc aac ggc gca cga gtc caa cgc gtg     192
   Leu Gly Val Val Asp Asn Asn Gly Asn Gly Ala Arg Val Gln Arg Val
       50                  55                  60
   gtc ggg agc gct ccg gcg gca agt ctc ggc atc tcc acc ggc gac gtg     240
   Val Gly Ser Ala Pro Ala Ala Ser Leu Gly Ile Ser Thr Gly Asp Val
   65                  70                  75                  80
   atc acc gcg gtc gac ggc gct ccg atc aac tcg gcc acc gcg atg gcg     288
   Ile Thr Ala Val Asp Gly Ala Pro Ile Asn Ser Ala Thr Ala Met Ala
                   85                  90                  95
   gac gcg ctt aac ggg cat cat ccc ggt gac gtc atc tcg gtg acc tgg     336
   Asp Ala Leu Asn Gly His His Pro Gly Asp Val Ile Ser Val Thr Trp
               100                 105                 110
   caa acc aag tcg ggc ggc acg cgt aca ggg aac gtg aca ttg gcc gag     384
   Gln Thr Lys Ser Gly Gly Thr Arg Thr Gly Asn Val Thr Leu Ala Glu
           115                 120                 125
   gga ccc ccg gcc                                                     396
   Gly Pro Pro Ala
       130
<210> 4
   <211> 132
   <212> PRT
   <213> Mycobacterium tuberculosis
   <223> 14 KD C-terminal fragment of MTB32A Ra12
<400> 4
   Thr Ala Ala Ser Asp Asn Phe Gln Leu Ser Gln Gly Gly Gln Gly Phe
    1               5                  10                  15
   Ala Ile Pro Ile Gly Gln Ala Met Ala Ile Ala Gly Gln Ile Arg Ser
               20                  25                  30
   Gly Gly Gly Ser Pro Thr Val His Ile Gly Pro Thr Ala Phe Leu Gly
           35                  40                  45
   Leu Gly Val Val Asp Asn Asn Gly Asn Gly Ala Arg Val Gln Arg Val
       50                  55                  60
   Val Gly Ser Ala Pro Ala Ala Ser Leu Gly Ile Ser Thr Gly Asp Val
   65                  70                  75                  80
   Ile Thr Ala Val Asp Gly Ala Pro Ile Asn Ser Ala Thr Ala Met Ala
                   85                  90                  95
   Asp Ala Leu Asn Gly His His Pro Gly Asp Val Ile Ser Val Thr Trp
               100                 105                 110
   Gln Thr Lys Ser Gly Gly Thr Arg Thr Gly Asn Val Thr Leu Ala Glu
           115                 120                 125
   Gly Pro Pro Ala
       130
<210> 5
   <211> 702
   <212> DNA
   <213> Artificial Sequence
<220>
   <221> CDS
   <222> (4)..(696)
   <223> Ra12-DPPD fusion polypeptide
<220>
   <223> Description of Artificial Sequence:Ra12-DPPD fusion polypeptide
<400> 5
   cat atg cat cac cat cac cat cac acg gcc gcg tcc gat aac ttc cag     48
       Met His His His His His His Thr Ala Ala Ser Asp Asn Phe Gln
        1               5                  10                  15
   ctg tcc cag ggt ggg cag gga ttc gcc att ccg atc ggg cag gcg atg     96
   Leu Ser Gln Gly Gly Gln Gly Phe Ala Ile Pro Ile Gly Gln Ala Met
                   20                  25                  30
   gcg atc gcg ggc cag atc cga tcg ggt ggg ggg tca ccc acc gtt cat    144
   Ala Ile Ala Gly Gln Ile Arg Ser Gly Gly Gly Ser Pro Thr Val His
               35                  40                  45
   atc ggg cct acc gcc ttc ctc ggc ttg ggt gtt gtc gac aac aac ggc    192
   Ile Gly Pro Thr Ala Phe Leu Gly Leu Gly Val Val Asp Asn Asn Gly
           50                  55                  60
   aac ggc gca cga gtc caa cgc gtg gtc ggg agc gct ccg gcg gca agt    240
   Asn Gly Ala Arg Val Gln Arg Val Val Gly Ser Ala Pro Ala Ala Ser
       65                  70                  75
   ctc ggc atc tcc acc ggc gac gtg atc acc gcg gtc gac ggc gct ccg    288
   Leu Gly Ile Ser Thr Gly Asp Val Ile Thr Ala Val Asp Gly Ala Pro
   80                  85                  90                  95
   atc aac tcg gcc acc gcg atg gcg gac gcg ctt aac ggg cat cat ccc    336
   Ile Asn Ser Ala Thr Ala Met Ala Asp Ala Leu Asn Gly His His Pro
                   100                 105                 110
   ggt gac gtc atc tcg gtg acc tgg caa acc aag tcg ggc ggc acg cgt    384
   Gly Asp Val Ile Ser Val Thr Trp Gln Thr Lys Ser Gly Gly Thr Arg
               115                 120                 125
   aca ggg aac gtg aca ttg gcc gag gga ccc ccg gcc gaa ttc gac gac    432
   Thr Gly Asn Val Thr Leu Ala Glu Gly Pro Pro Ala Glu Phe Asp Asp
           130                 135                 140
   gac gac aag gat cca cct gac ccg cat cag ccg gac atg acg aaa ggc    480
   Asp Asp Lys Asp Pro Pro Asp Pro His Gln Pro Asp Met Thr Lys Gly
       145                 150                 155
   tat tgc ccg ggt ggc cga tgg ggt ttt ggc gac ttg gcc gtg tgc gac    528
   Tyr Cys Pro Gly Gly Arg Trp Gly Phe Gly Asp Leu Ala Val Cys Asp
   160                 165                 170                 175
   ggc gag aag tac ccc gac ggc tcg ttt tgg cac cag tgg atg caa acg    576
   Gly Glu Lys Tyr Pro Asp Gly Ser Phe Trp His Gln Trp Met Gln Thr
                   180                 185                 190
   tgg ttt acc ggc cca cag ttt tac ttc gat tgt gtc agc ggc ggt gag    624
   Trp Phe Thr Gly Pro Gln Phe Tyr Phe Asp Cys Val Ser Gly Gly Glu
               195                 200                 205
   ccc ctc ccc ggc ccg ccg cca ccg ggt ggt tgc ggt ggg gca att ccg    672
   Pro Leu Pro Gly Pro Pro Pro Pro Gly Gly Cys Gly Gly Ala Ile Pro
   210 215 220
   tcc gag cag ccc aac gct ccc tga gaattc 702
   Ser Glu Gln Pro Asn Ala Pro
   225 230
<210> 6
   <211> 230
   <212> PRT
   <213> Artificial Sequence
   <223> Description of Artificial Sequence:Ra12-DPPD fusion polypeptide
<400> 6
   Met His His His His His His Thr Ala Ala Ser Asp Asn Phe Gln Leu
   1 5 10 15
   Ser Gln Gly Gly Gln Gly Phe Ala Ile Pro Ile Gly Gln Ala Met Ala
   20 25 30
   Ile Ala Gly Gln Ile Arg Ser Gly Gly Gly Ser Pro Thr Val His Ile
   35 40 45
   Gly Pro Thr Ala Phe Leu Gly Leu Gly Val Val Asp Asn Asn Gly Asn
   50 55 60
   Gly Ala Arg Val Gln Arg Val Val Gly Ser Ala Pro Ala Ala Ser Leu
   65 70 75 80
   Gly Ile Ser Thr Gly Asp Val Ile Thr Ala Val Asp Gly Ala Pro Ile
   85 90 95
   Asn Ser Ala Thr Ala Met Ala Asp Ala Leu Asn Gly His His Pro Gly
   100 105 110
   Asp Val Ile Ser Val Thr Trp Gln Thr Lys Ser Gly Gly Thr Arg Thr
   115 120 125
   Gly Asn Val Thr Leu Ala Glu Gly Pro Pro Ala Glu Phe Asp Asp Asp
   130 135 140
   Asp Lys Asp Pro Pro Asp Pro His Gln Pro Asp Met Thr Lys Gly Tyr
   145 150 155 160
   Cys Pro Gly Gly Arg Trp Gly Phe Gly Asp Leu Ala Val Cys Asp Gly
   165 170 175
   Glu Lys Tyr Pro Asp Gly Ser Phe Trp His Gln Trp Met Gln Thr Trp
   180 185 190
   Phe Thr Gly Pro Gln Phe Tyr Phe Asp Cys Val Ser Gly Gly Glu Pro
   195 200 205
   Leu Pro Gly Pro Pro Pro Pro Gly Gly Cys Gly Gly Ala Ile Pro Ser
   210 215 220
   Glu Gln Pro Asn Ala Pro
   225 230
<210> 7
   <211> 1746
   <212> DNA
   <213> Artificial Sequence
<220>
   <223> Description of Artificial Sequence:Ra12-WT1 fusion
<220>
   <221> CDS
   <222> (4)..(1740)
   <223> Ra12-WT1 fusion polypeptide
<400> 7
   cat atg cat cac cat cac cat cac acg gcc gcg tcc gat aac ttc cag     48
       Met His His His His His His Thr Ala Ala Ser Asp Asn Phe Gln
        1               5                  10                  15
   ctg tcc cag ggt ggg cag gga ttc gcc att ccg atc ggg cag gcg atg     96
   Leu Ser Gln Gly Gly Gln Gly Phe Ala Ile Pro Ile Gly Gln Ala Met
                   20                  25                  30
   gcg atc gcg ggc cag atc cga tcg ggt ggg ggg tca ccc acc gtt cat    144
   Ala Ile Ala Gly Gln Ile Arg Ser Gly Gly Gly Ser Pro Thr Val His
               35                  40                  45
   atc ggg cct acc gcc ttc ctc ggc ttg ggt gtt gtc gac aac aac ggc    192
   Ile Gly Pro Thr Ala Phe Leu Gly Leu Gly Val Val Asp Asn Asn Gly
           50                  55                  60
   aac ggc gca cga gtc caa cgc gtg gtc ggg agc gct ccg gcg gca agt    240
   Asn Gly Ala Arg Val Gln Arg Val Val Gly Ser Ala Pro Ala Ala Ser
       65                  70                  75
   ctc ggc atc tcc acc ggc gac gtg atc acc gcg gtc gac ggc gct ccg    288
   Leu Gly Ile Ser Thr Gly Asp Val Ile Thr Ala Val Asp Gly Ala Pro
   80                  85                  90                  95
   atc aac tcg gcc acc gcg atg gcg gac gcg ctt aac ggg cat cat ccc    336
   Ile Asn Ser Ala Thr Ala Met Ala Asp Ala Leu Asn Gly His His Pro
                   100                 105                 110
   ggt gac gtc atc tcg gtg acc tgg caa acc aag tcg ggc ggc acg cgt    384
   Gly Asp Val Ile Ser Val Thr Trp Gln Thr Lys Ser Gly Gly Thr Arg
               115                 120                 125
   aca ggg aac gtg aca ttg gcc gag gga ccc ccg gcc gaa ttc ccg ctg    432
   Thr Gly Asn Val Thr Leu Ala Glu Gly Pro Pro Ala Glu Phe Pro Leu
           130                 135                 140
   gtg ccg cgc ggc agc ccg atg ggc tcc gac gtt cgg gac ctg aac gca    480
   Val Pro Arg Gly Ser Pro Met Gly Ser Asp Val Arg Asp Leu Asn Ala
       145                 150                 155
   ctg ctg ccg gca gtt ccg tcc ctg ggt ggt ggt ggt ggt tgc gca ctg    528
   Leu Leu Pro Ala Val Pro Ser Leu Gly Gly Gly Gly Gly Cys Ala Leu
   160                 165                 170                 175
   ccg gtt agc ggt gca gca cag tgg gct ccg gtt ctg gac ttc gca ccg    576
   Pro Val Ser Gly Ala Ala Gln Trp Ala Pro Val Leu Asp Phe Ala Pro
                   180                 185                 190
   ccg ggt gca tcc gca tac ggt tcc ctg ggt ggt ccg gca ccg ccg ccg    624
   Pro Gly Ala Ser Ala Tyr Gly Ser Leu Gly Gly Pro Ala Pro Pro Pro
               195                 200                 205
   gca ccg ccg ccg ccg ccg ccg ccg ccg ccg cac tcc ttc atc aaa cag    672
   Ala Pro Pro Pro Pro Pro Pro Pro Pro Pro His Ser Phe Ile Lys Gln
           210                 215                 220
   gaa ccg agc tgg ggt ggt gca gaa ccg cac gaa gaa cag tgc ctg agc    720
   Glu Pro Ser Trp Gly Gly Ala Glu Pro His Glu Glu Gln Cys Leu Ser
       225                 230                 235
   gca ttc acc gtt cac ttc tcc ggc cag ttc act ggc aca gcc gga gcc    768
   Ala Phe Thr Val His Phe Ser Gly Gln Phe Thr Gly Thr Ala Gly Ala
   240                 245                 250                 255
   tgt cgc tac ggg ccc ttc ggt cct cct ccg ccc agc cag gcg tca tcc    816
   Cys Arg Tyr Gly Pro Phe Gly Pro Pro Pro Pro Ser Gln Ala Ser Ser
                   260                 265                 270
   ggc cag gcc agg atg ttt cct aac gcg ccc tac ctg ccc agc tgc ctc    864
   Gly Gln Ala Arg Met Phe Pro Asn Ala Pro Tyr Leu Pro Ser Cys Leu
               275                 280                 285
   gag agc cag ccc gct att cgc aat cag ggt tac agc acg gtc acc ttc    912
   Glu Ser Gln Pro Ala Ile Arg Asn Gln Gly Tyr Ser Thr Val Thr Phe
           290                 295                 300
   gac ggg acg ccc agc tac ggt cac acg ccc tcg cac cat gcg gcg cag    960
   Asp Gly Thr Pro Ser Tyr Gly His Thr Pro Ser His His Ala Ala Gln
       305                 310                 315
   ttc ccc aac cac tca ttc aag cat gag gat ccc atg ggc cag cag ggc    1008
   Phe Pro Asn His Ser Phe Lys His Glu Asp Pro Met Gly Gln Gln Gly
   320                 325                 330                 335
   tcg ctg ggt gag cag cag tac tcg gtg ccg ccc ccg gtc tat ggc tgc    1056
   Ser Leu Gly Glu Gln Gln Tyr Ser Val Pro Pro Pro Val Tyr Gly Cys
                   340                 345                 350
   cac acc ccc acc gac agc tgc acc ggc agc cag gct ttg ctg ctg agg    1104
   His Thr Pro Thr Asp Ser Cys Thr Gly Ser Gln Ala Leu Leu Leu Arg
               355                 360                 365
   acg ccc tac agc agt gac aat tta tac caa atg aca tcc cag ctt gaa    1152
   Thr Pro Tyr Ser Ser Asp Asn Leu Tyr Gln Met Thr Ser Gln Leu Glu
           370                 375                 380
   tgc atg acc tgg aat cag atg aac tta gga gcc acc tta aag ggc cac    1200
   Cys Met Thr Trp Asn Gln Met Asn Leu Gly Ala Thr Leu Lys Gly His
       385                 390                 395
   agc aca ggg tac gag agc gat aac cac aca acg ccc atc ctc tgc gga    1248
   Ser Thr Gly Tyr Glu Ser Asp Asn His Thr Thr Pro Ile Leu Cys Gly
   400                 405                 410                 415
   gcc caa tac aga ata cac acg cac ggt gtc ttc aga ggc att cag gat    1296
   Ala Gln Tyr Arg Ile His Thr His Gly Val Phe Arg Gly Ile Gln Asp
                   420                 425                 430
   gtg cga cgt gtg cct gga gta gcc ccg act ctt gta cgg tcg gca tct    1344
   Val Arg Arg Val Pro Gly Val Ala Pro Thr Leu Val Arg Ser Ala Ser
               435                 440                 445
   gag acc agt gag aaa cgc ccc ttc atg tgt gct tac tca ggc tgc aat    1392
   Glu Thr Ser Glu Lys Arg Pro Phe Met Cys Ala Tyr Ser Gly Cys Asn
           450                 455                 460
   aag aga tat ttt aag ctg tcc cac tta cag atg cac agc agg aag cac    1440
   Lys Arg Tyr Phe Lys Leu Ser His Leu Gln Met His Ser Arg Lys His
       465                 470                 475
   act ggt gag aaa cca tac cag tgt gac ttc aag gac tgt gaa cga agg    1488
   Thr Gly Glu Lys Pro Tyr Gln Cys Asp Phe Lys Asp Cys Glu Arg Arg
   480                     485                     490                     495
   ttt ttt cgt tca gac cag ctc aaa aga cac caa agg aga cat aca ggt        1536
   Phe Phe Arg Ser Asp Gln Leu Lys Arg His Gln Arg Arg His Thr Gly
                   500                     505                     510
   gtg aaa cca ttc cag tgt aaa act tgt cag cga aag ttc tcc cgg tcc        1584
   Val Lys Pro Phe Gln Cys Lys Thr Cys Gln Arg Lys Phe Ser Arg Ser
               515                     520                     525
   gac cac ctg aag acc cac acc agg act cat aca ggt gaa aag ccc ttc        1632
   Asp His Leu Lys Thr His Thr Arg Thr His Thr Gly Glu Lys Pro Phe
           530                     535                     540
   agc tgt cgg tgg cca agt tgt cag aaa aag ttt gcc cgg tca gat gaa        1680
   Ser Cys Arg Trp Pro Ser Cys Gln Lys Lys Phe Ala Arg Ser Asp Glu
       545                     550                     555
   tta gtc cgc cat cac aac atg cat cag aga aac atg acc aaa ctc cag        1728
   Leu Val Arg His His Asn Met His Gln Arg Asn Met Thr Lys Leu Gln
   560                     565                     570                     575
   ctg gcg ctt tga gaattc                                                 1746
   Leu Ala Leu
<210> 8
   <211> 578
   <212> PRT
   <213> Artificial Sequence
   <223> Description of Artificial Sequence:Ra12-WT1 fusion polypeptide
<400> 8
   Met His His His His His His Thr Ala Ala Ser Asp Asn Phe Gln Leu
    1               5                  10                  15
   Ser Gln Gly Gly Gln Gly Phe Ala Ile Pro Ile Gly Gln Ala Met Ala
               20                  25                  30
   Ile Ala Gly Gln Ile Arg Ser Gly Gly Gly Ser Pro Thr Val His Ile
           35                  40                  45
   Gly Pro Thr Ala Phe Leu Gly Leu Gly Val Val Asp Asn Asn Gly Asn
       50                  55                  60
   Gly Ala Arg Val Gln Arg Val Val Gly Ser Ala Pro Ala Ala Ser Leu
   65                  70                  75                  80
   Gly Ile Ser Thr Gly Asp Val Ile Thr Ala Val Asp Gly Ala Pro Ile
                   85                  90                  95
   Asn Ser Ala Thr Ala Met Ala Asp Ala Leu Asn Gly His His Pro Gly
               100                 105                 110
   Asp Val Ile Ser Val Thr Trp Gln Thr Lys Ser Gly Gly Thr Arg Thr
           115                 120                 125
   Gly Asn Val Thr Leu Ala Glu Gly Pro Pro Ala Glu Phe Pro Leu Val
       130                 135                 140
   Pro Arg Gly Ser Pro Met Gly Ser Asp Val Arg Asp Leu Asn Ala Leu
   145                 150                 155                 160
   Leu Pro Ala Val Pro Ser Leu Gly Gly Gly Gly Gly Cys Ala Leu Pro
                   165                 170                 175
   Val Ser Gly Ala Ala Gln Trp Ala Pro Val Leu Asp Phe Ala Pro Pro
               180                 185                 190
   Gly Ala Ser Ala Tyr Gly Ser Leu Gly Gly Pro Ala Pro Pro Pro Ala
           195                 200                 205
   Pro Pro Pro Pro Pro Pro Pro Pro Pro His Ser Phe Ile Lys Gln Glu
           210                 215                 220
   Pro Ser Trp Gly Gly Ala Glu Pro His Glu Glu Gln Cys Leu Ser Ala
   225                 230                 235                 240
   Phe Thr Val His Phe Ser Gly Gln Phe Thr Gly Thr Ala Gly Ala Cys
                   245                 250                 255
   Arg Tyr Gly Pro Phe Gly Pro Pro Pro Pro Ser Gln Ala Ser Ser Gly
               260                 265                 270
   Gln Ala Arg Met Phe Pro Asn Ala Pro Tyr Leu Pro Ser Cys Leu Glu
           275                 280                 285
   Ser Gln Pro Ala Ile Arg Asn Gln Gly Tyr Ser Thr Val Thr Phe Asp
       290                 295                 300
   Gly Thr Pro Ser Tyr Gly His Thr Pro Ser His His Ala Ala Gln Phe
   305                 310                 315                 320
   Pro Asn His Ser Phe Lys His Glu Asp Pro Met Gly Gln Gln Gly Ser
                   325                 330                 335
   Leu Gly Glu Gln Gln Tyr Ser Val Pro Pro Pro Val Tyr Gly Cys His
               340                 345                 350
   Thr Pro Thr Asp Ser Cys Thr Gly Ser Gln Ala Leu Leu Leu Arg Thr
           355                 360                 365
   Pro Tyr Ser Ser Asp Asn Leu Tyr Gln Met Thr Ser Gln Leu Glu Cys
       370                 375                 380
   Met Thr Trp Asn Gln Met Asn Leu Gly Ala Thr Leu Lys Gly His Ser
   385                 390                 395                 400
   Thr Gly Tyr Glu Ser Asp Asn His Thr Thr Pro Ile Leu Cys Gly Ala
                   405                 410                 415
   Gln Tyr Arg Ile His Thr His Gly Val Phe Arg Gly Ile Gln Asp Val
               420                 425                 430
   Arg Arg Val Pro Gly Val Ala Pro Thr Leu Val Arg Ser Ala Ser Glu
           435                 440                 445
   Thr Ser Glu Lys Arg Pro Phe Met Cys Ala Tyr Ser Gly Cys Asn Lys
       450                 455                 460
   Arg Tyr Phe Lys Leu Ser His Leu Gln Met His Ser Arg Lys His Thr
   465                 470                 475                 480
   Gly Glu Lys Pro Tyr Gln Cys Asp Phe Lys Asp Cys Glu Arg Arg Phe
                   485                 490                 495
   Phe Arg Ser Asp Gln Leu Lys Arg His Gln Arg Arg His Thr Gly Val
               500                 505                 510
   Lys Pro Phe Gln Cys Lys Thr Cys Gln Arg Lys Phe Ser Arg Ser Asp
           515                 520                 525
   His Leu Lys Thr His Thr Arg Thr His Thr Gly Glu Lys Pro Phe Ser
       530                 535                 540
   Cys Arg Trp Pro Ser Cys Gln Lys Lys Phe Ala Arg Ser Asp Glu Leu
   545                 550                 555                 560
   Val Arg His His Asn Met His Gln Arg Asn Met Thr Lys Leu Gln Leu
                   565                 570                 575
   Ala Leu
<210> 9
   <211> 672
   <212> DNA
   <213> Artificial Sequence
<220>
   <223> Description of Artificial Sequence:Ra12-human mammaglobin fusion
<220>
   <221> CDS
   <222> (4)..(666)
   <223> Ra12-human mammaglobin fusion polypeptide
<400> 9
   cat atg cat cac cat cac cat cac acg gcc gcg tcc gat aac ttc cag      48
   ctg tcc cag ggt ggg cag gga ttc gcc att ccg atc ggg cag gcg atg      96
   gcg atc gcg ggc cag atc cga tcg ggt ggg ggg tca ccc acc gtt cat     144
   atc ggg cct acc gcc ttc ctc ggc ttg ggt gtt gtc gac aac aac ggc     192
   aac ggc gca cga gtc caa cgc gtg gtc ggg agc gct ccg gcg gca agt     240
   ctc ggc atc tcc acc ggc gac gtg atc acc gcg gtc gac ggc gct ccg     288
   atc aac tcg gcc acc gcg atg gcg gac gcg ctt aac ggg cat cat ccc     336
   ggt gac gtc atc tcg gtg acc tgg caa acc aag tcg ggc ggc acg cgt     384
   aca ggg aac gtg aca ttg gcc gag gga ccc ccg gcc gaa ttc atc gag     432
   gga agg ggc tct ggc tgc ccc tta ttg gag aat gtg att tcc aag aca     480
   atc aat cca caa gtg tct aag act gaa tac aaa gaa ctt ctt caa gag     528
   ttc ata gac gac aat gcc act aca aat gcc ata gat gaa ttg aag gaa     576
   tgt ttt ctt aac caa acg gat gaa act ctg agc aat gtt gag gtg ttt     624
   atg caa tta ata tat gac agc agt ctt tgt gat tta ttt taa gaattc      672
<210> 10
   <211> 220
   <212> PRT
   <213> Artificial Sequence
   <223> Description of Artificial Sequence:Ra12-human mammaglobin fusion polypeptide
<400> 10
<210> 11
   <211> 2191
   <212> DNA
   <213> Artificial Sequence
<220>
   <223> Description of Artificial Sequence:Ra12-H9-32A fusion (Ra12-MTB39-MTB32A(N-ter) fusion)
<220>
   <221> CDS
   <222> (1)..(2190)
   <223> Ra12-H9-32A (Ra12-MTH39-MTH32A(N-ter)) fusion polypeptide
<400> 11
   atg cat cac cat cac cat cac acg gcc gcg tcc gat aac ttc cag ctg    48
   Met His His His His His His Thr Ala Ala Ser Asp Asn Phe Gln Leu
    1               5                  10                  15
   tcc cag ggt ggg cag gga ttc gcc att ccg atc ggg cag gcg atg gcg    96
   Ser Gln Gly Gly Gln Gly Phe Ala Ile Pro Ile Gly Gln Ala Met Ala
               20                  25                  30
   atc gcg ggc cag atc cga tcg ggt ggg ggg tca ccc acc gtt cat atc    144
   Ile Ala Gly Gln Ile Arg Ser Gly Gly Gly Ser Pro Thr Val His Ile
           35                  40                  45
   ggg cct acc gcc ttc ctc ggc ttg ggt gtt gtc gac aac aac ggc aac     192
   Gly Pro Thr Ala Phe Leu Gly Leu Gly Val Val Asp Asn Asn Gly Asn
       50                  55                  60
   ggc gca cga gtc caa cgc gtg gtc ggg agc gct ccg gcg gca agt ctc     240
   Gly Ala Arg Val Gln Arg Val Val Gly Ser Ala Pro Ala Ala Ser Leu
   65                  70                  75                  80
   ggc atc tcc acc ggc gac gtg atc acc gcg gtc gac ggc gct ccg atc     288
   Gly Ile Ser Thr Gly Asp Val Ile Thr Ala Val Asp Gly Ala Pro Ile
                   85                  90                  95
   aac tcg gcc acc gcg atg gcg gac gcg ctt aac ggg cat cat ccc ggt     336
   Asn Ser Ala Thr Ala Met Ala Asp Ala Leu Asn Gly His His Pro Gly
               100                 105                 110
   gac gtc atc tcg gtg acc tgg caa acc aag tcg ggc ggc acg cgt aca     384
   Asp Val Ile Ser Val Thr Trp Gln Thr Lys Ser Gly Gly Thr Arg Thr
           115                 120                 125
   ggg aac gtg aca ttg gcc gag gga ccc ccg gcc gaa ttc atg gtg gat     432
   Gly Asn Val Thr Leu Ala Glu Gly Pro Pro Ala Glu Phe Met Val Asp
       130                 135                 140
   ttc ggg gcg tta cca ccg gag atc aac tcc gcg agg atg tac gcc ggc     480
   Phe Gly Ala Leu Pro Pro Glu Ile Asn Ser Ala Arg Met Tyr Ala Gly
   145                 150                 155                 160
   ccg ggt tcg gcc tcg ctg gtg gcc gcg gct cag atg tgg gac agc gtg     528
   Pro Gly Ser Ala Ser Leu Val Ala Ala Ala Gln Met Trp Asp Ser Val
                   165                 170                 175
   gcg agt gac ctg ttt tcg gcc gcg tcg gcg ttt cag tcg gtg gtc tgg     576
   Ala Ser Asp Leu Phe Ser Ala Ala Ser Ala Phe Gln Ser Val Val Trp
               180                 185                 190
   ggt ctg acg gtg ggg tcg tgg ata ggt tcg tcg gcg ggt ctg atg gtg     624
   Gly Leu Thr Val Gly Ser Trp Ile Gly Ser Ser Ala Gly Leu Met Val
           195                 200                 205
   gcg gcg gcc tcg ccg tat gtg gcg tgg atg agc gtc acc gcg ggg cag     672
   Ala Ala Ala Ser Pro Tyr Val Ala Trp Met Ser Val Thr Ala Gly Gln
       210                 215                 220
   gcc gag ctg acc gcc gcc cag gtc cgg gtt gct gcg gcg gcc tac gag     720
   Ala Glu Leu Thr Ala Ala Gln Val Arg Val Ala Ala Ala Ala Tyr Glu
   225                 230                 235                 240
   acg gcg tat ggg ctg acg gtg ccc ccg ccg gtg atc gcc gag aac cgt     768
   Thr Ala Tyr Gly Leu Thr Val Pro Pro Pro Val Ile Ala Glu Asn Arg
                   245                 250                 255
   gct gaa ctg atg att ctg ata gcg acc aac ctc ttg ggg caa aac acc     816
   Ala Glu Leu Met Ile Leu Ile Ala Thr Asn Leu Leu Gly Gln Asn Thr
               260                 265                 270
   ccg gcg atc gcg gtc aac gag gcc gaa tac ggc gag atg tgg gcc caa     864
   Pro Ala Ile Ala Val Asn Glu Ala Glu Tyr Gly Glu Met Trp Ala Gln
           275                 280                 285
   gac gcc gcc gcg atg ttt ggc tac gcc gcg gcg acg gcg acg gcg acg      912
   Asp Ala Ala Ala Met Phe Gly Tyr Ala Ala Ala Thr Ala Thr Ala Thr
       290                 295                 300
   gcg acg ttg ctg ccg ttc gag gag gcg ccg gag atg acc agc gcg ggt      960
   Ala Thr Leu Leu Pro Phe Glu Glu Ala Pro Glu Met Thr Ser Ala Gly
   305                 310                 315                 320
   ggg ctc ctc gag cag gcc gcc gcg gtc gag gag gcc tcc gac acc gcc     1008
   Gly Leu Leu Glu Gln Ala Ala Ala Val Glu Glu Ala Ser Asp Thr Ala
                   325                 330                 335
   gcg gcg aac cag ttg atg aac aat gtg ccc cag gcg ctg caa cag ctg     1056
   Ala Ala Asn Gln Leu Met Asn Asn Val Pro Gln Ala Leu Gln Gln Leu
               340                 345                 350
   gcc cag ccc acg cag ggc acc acg cct tct tcc aag ctg ggt ggc ctg     1104
   Ala Gln Pro Thr Gln Gly Thr Thr Pro Ser Ser Lys Leu Gly Gly Leu
           355                 360                 365
   tgg aag acg gtc tcg ccg cat cgg tcg ccg atc agc aac atg gtg tcg     1152
   Trp Lys Thr Val Ser Pro His Arg Ser Pro Ile Ser Asn Met Val Ser
       370                 375                 380
   atg gcc aac aac cac atg tcg atg acc aac tcg ggt gtg tcg atg acc     1200
   Met Ala Asn Asn His Met Ser Met Thr Asn Ser Gly Val Ser Met Thr
   385                 390                 395                 400
   aac acc ttg agc tcg atg ttg aag ggc ttt gct ccg gcg gcg gcc gcc     1248
   Asn Thr Leu Ser Ser Met Leu Lys Gly Phe Ala Pro Ala Ala Ala Ala
                   405                 410                 415
   cag gcc gtg caa acc gcg gcg caa aac ggg gtc cgg gcg atg agc tcg     1296
   Gln Ala Val Gln Thr Ala Ala Gln Asn Gly Val Arg Ala Met Ser Ser
               420                 425                 430
   ctg ggc agc tcg ctg ggt tct tcg ggt ctg ggc ggt ggg gtg gcc gcc     1344
   Leu Gly Ser Ser Leu Gly Ser Ser Gly Leu Gly Gly Gly Val Ala Ala
           435                 440                 445
   aac ttg ggt cgg gcg gcc tcg gtc ggt tcg ttg tcg gtg ccg cag gcc     1392
   Asn Leu Gly Arg Ala Ala Ser Val Gly Ser Leu Ser Val Pro Gln Ala
       450                 455                 460
   tgg gcc gcg gcc aac cag gca gtc acc ccg gcg gcg cgg gcg ctg ccg     1440
   Trp Ala Ala Ala Asn Gln Ala Val Thr Pro Ala Ala Arg Ala Leu Pro
   465                 470                 475                 480
   ctg acc agc ctg acc agc gcc gcg gaa aga ggg ccc ggg cag atg ctg     1488
   Leu Thr Ser Leu Thr Ser Ala Ala Glu Arg Gly Pro Gly Gln Met Leu
                   485                 490                 495
   ggc ggg ctg ccg gtg ggg cag atg ggc gcc agg gcc ggt ggt ggg ctc     1536
   Gly Gly Leu Pro Val Gly Gln Met Gly Ala Arg Ala Gly Gly Gly Leu
               500                 505                 510
   agt ggt gtg ctg cgt gtt ccg ccg cga ccc tat gtg atg ccg cat tct     1584
   Ser Gly Val Leu Arg Val Pro Pro Arg Pro Tyr Val Met Pro His Ser
           515                 520                 525
   ccg gca gcc ggc gat atc gcc ccg ccg gcc ttg tcg cag gac cgg ttc     1632
   Pro Ala Ala Gly Asp Ile Ala Pro Pro Ala Leu Ser Gln Asp Arg Phe
       530                 535                 540
   gcc gac ttc ccc gcg ctg ccc ctc gac ccg tcc gcg atg gtc gcc caa     1680
   Ala Asp Phe Pro Ala Leu Pro Leu Asp Pro Ser Ala Met Val Ala Gln
   545                 550                 555                 560
   gtg ggg cca cag gtg gtc aac atc aac acc aaa ctg ggc tac aac aac     1728
   Val Gly Pro Gln Val Val Asn Ile Asn Thr Lys Leu Gly Tyr Asn Asn
                   565                 570                 575
   gcc gtg ggc gcc ggg acc ggc atc gtc atc gat ccc aac ggt gtc gtg     1776
   Ala Val Gly Ala Gly Thr Gly Ile Val Ile Asp Pro Asn Gly Val Val
               580                 585                 590
   ctg acc aac aac cac gtg atc gcg ggc gcc acc gac atc aat gcg ttc     1824
   Leu Thr Asn Asn His Val Ile Ala Gly Ala Thr Asp Ile Asn Ala Phe
           595                 600                 605
   agc gtc ggc tcc ggc caa acc tac ggc gtc gat gtg gtc ggg tat gac     1872
   Ser Val Gly Ser Gly Gln Thr Tyr Gly Val Asp Val Val Gly Tyr Asp
       610                 615                 620
   cgc acc cag gat gtc gcg gtg ctg cag ctg cgc ggt gcc ggt ggc ctg     1920
   Arg Thr Gln Asp Val Ala Val Leu Gln Leu Arg Gly Ala Gly Gly Leu
   625                 630                 635                 640
   ccg tcg gcg gcg atc ggt ggc ggc gtc gcg gtt ggt gag ccc gtc gtc     1968
   Pro Ser Ala Ala Ile Gly Gly Gly Val Ala Val Gly Glu Pro Val Val
                   645                 650                 655
   gcg atg ggc aac agc ggt ggg cag ggc gga acg ccc cgt gcg gtg cct     2016
   Ala Met Gly Asn Ser Gly Gly Gln Gly Gly Thr Pro Arg Ala Val Pro
               660                 665                 670
   ggc agg gtg gtc gcg ctc ggc caa acc gtg cag gcg tcg gat tcg ctg     2064
   Gly Arg Val Val Ala Leu Gly Gln Thr Val Gln Ala Ser Asp Ser Leu
           675                 680                 685
   acc ggt gcc gaa gag aca ttg aac ggg ttg atc cag ttc gat gcc gcg     2112
   Thr Gly Ala Glu Glu Thr Leu Asn Gly Leu Ile Gln Phe Asp Ala Ala
       690                 695                 700
   atc cag ccc ggt gat tcg ggc ggg ccc gtc gtc aac ggc cta gga cag     2160
   Ile Gln Pro Gly Asp Ser Gly Gly Pro Val Val Asn Gly Leu Gly Gln
   705                 710                 715                 720
   gtg gtc ggt atg aac acg gcc gcg tcc tag g                           2191
   Val Val Gly Met Asn Thr Ala Ala Ser
                   725                 730
<210> 12
   <211> 729
   <212> PRT
   <213> Artificial Sequence
   <223> Description of Artificial Sequence:Ra12-H9-32A fusion polypeptide (Ra12-MTB39-MTB32A(N-ter) fusion polypeptide)
<400> 12
   Met His His His His His His Thr Ala Ala Ser Asp Asn Phe Gln Leu
    1               5                  10                  15
   Ser Gln Gly Gly Gln Gly Phe Ala Ile Pro Ile Gly Gln Ala Met Ala
               20                  25                  30
   Ile Ala Gly Gln Ile Arg Ser Gly Gly Gly Ser Pro Thr Val His Ile
           35                  40                  45
   Gly Pro Thr Ala Phe Leu Gly Leu Gly Val Val Asp Asn Asn Gly Asn
       50                  55                  60
   Gly Ala Arg Val Gln Arg Val Val Gly Ser Ala Pro Ala Ala Ser Leu
   65                  70                  75                  80
   Gly Ile Ser Thr Gly Asp Val Ile Thr Ala Val Asp Gly Ala Pro Ile
                   85                  90                  95
   Asn Ser Ala Thr Ala Met Ala Asp Ala Leu Asn Gly His His Pro Gly
               100                 105                 110
   Asp Val Ile Ser Val Thr Trp Gln Thr Lys Ser Gly Gly Thr Arg Thr
           115                 120                 125
   Gly Asn Val Thr Leu Ala Glu Gly Pro Pro Ala Glu Phe Met Val Asp
       130                 135                 140
   Phe Gly Ala Leu Pro Pro Glu Ile Asn Ser Ala Arg Met Tyr Ala Gly
   145                 150                 155                 160
   Pro Gly Ser Ala Ser Leu Val Ala Ala Ala Gln Met Trp Asp Ser Val
                   165                 170                 175
   Ala Ser Asp Leu Phe Ser Ala Ala Ser Ala Phe Gln Ser Val Val Trp
               180                 185                 190
   Gly Leu Thr Val Gly Ser Trp Ile Gly Ser Ser Ala Gly Leu Met Val
           195                 200                 205
   Ala Ala Ala Ser Pro Tyr Val Ala Trp Met Ser Val Thr Ala Gly Gln
       210                 215                 220
   Ala Glu Leu Thr Ala Ala Gln Val Arg Val Ala Ala Ala Ala Tyr Glu
   225                 230                 235                 240
   Thr Ala Tyr Gly Leu Thr Val Pro Pro Pro Val Ile Ala Glu Asn Arg
                   245                 250                 255
   Ala Glu Leu Met Ile Leu Ile Ala Thr Asn Leu Leu Gly Gln Asn Thr
               260                 265                 270
   Pro Ala Ile Ala Val Asn Glu Ala Glu Tyr Gly Glu Met Trp Ala Gln
           275                 280                 285
   Asp Ala Ala Ala Met Phe Gly Tyr Ala Ala Ala Thr Ala Thr Ala Thr
       290                 295                 300
   Ala Thr Leu Leu Pro Phe Glu Glu Ala Pro Glu Met Thr Ser Ala Gly
   305                 310                 315                 320
   Gly Leu Leu Glu Gln Ala Ala Ala Val Glu Glu Ala Ser Asp Thr Ala
                   325                 330                 335
   Ala Ala Asn Gln Leu Met Asn Asn Val Pro Gln Ala Leu Gln Gln Leu
               340                 345                 350
   Ala Gln Pro Thr Gln Gly Thr Thr Pro Ser Ser Lys Leu Gly Gly Leu
           355                 360                 365
   Trp Lys Thr Val Ser Pro His Arg Ser Pro Ile Ser Asn Met Val Ser
       370                 375                 380
   Met Ala Asn Asn His Met Ser Met Thr Asn Ser Gly Val Ser Met Thr
   385                 390                 395                 400
   Asn Thr Leu Ser Ser Met Leu Lys Gly Phe Ala Pro Ala Ala Ala Ala
                   405                 410                 415
   Gln Ala Val Gln Thr Ala Ala Gln Asn Gly Val Arg Ala Met Ser Ser
               420                 425                 430
   Leu Gly Ser Ser Leu Gly Ser Ser Gly Leu Gly Gly Gly Val Ala Ala
           435                 440                 445
   Asn Leu Gly Arg Ala Ala Ser Val Gly Ser Leu Ser Val Pro Gln Ala
       450                 455                 460
   Trp Ala Ala Ala Asn Gln Ala Val Thr Pro Ala Ala Arg Ala Leu Pro
   465                 470                 475                 480
   Leu Thr Ser Leu Thr Ser Ala Ala Glu Arg Gly Pro Gly Gln Met Leu
                       485                     490                     495
   Gly Gly Leu Pro Val Gly Gln Met Gly Ala Arg Ala Gly Gly Gly Leu
                   500                     505                     510
   Ser Gly Val Leu Arg Val Pro Pro Arg Pro Tyr Val Met Pro His Ser
               515                     520                     525
   Pro Ala Ala Gly Asp Ile Ala Pro Pro Ala Leu Ser Gln Asp Arg Phe
           530                     535                     540
   Ala Asp Phe Pro Ala Leu Pro Leu Asp Pro Ser Ala Met Val Ala Gln
   545                     550                     555                     560
   Val Gly Pro Gln Val Val Asn Ile Asn Thr Lys Leu Gly Tyr Asn Asn
                       565                     570                     575
   Ala Val Gly Ala Gly Thr Gly Ile Val Ile Asp Pro Asn Gly Val Val
                   580                     585                     590
   Leu Thr Asn Asn His Val Ile Ala Gly Ala Thr Asp Ile Asn Ala Phe
               595                     600                     605
   Ser Val Gly Ser Gly Gln Thr Tyr Gly Val Asp Val Val Gly Tyr Asp
           610                     615                     620
   Arg Thr Gln Asp Val Ala Val Leu Gln Leu Arg Gly Ala Gly Gly Leu
   625                     630                     635                     640
   Pro Ser Ala Ala Ile Gly Gly Gly Val Ala Val Gly Glu Pro Val Val
                       645                     650                     655
   Ala Met Gly Asn Ser Gly Gly Gln Gly Gly Thr Pro Arg Ala Val Pro
                   660                     665                     670
   Gly Arg Val Val Ala Leu Gly Gln Thr Val Gln Ala Ser Asp Ser Leu
               675                     680                     685
   Thr Gly Ala Glu Glu Thr Leu Asn Gly Leu Ile Gln Phe Asp Ala Ala
           690                     695                     700
   Ile Gln Pro Gly Asp Ser Gly Gly Pro Val Val Asn Gly Leu Gly Gln
   705                     710                     715                     720
   Val Val Gly Met Asn Thr Ala Ala Ser
                       725
<210> 13
   <211> 51
   <212> DNA
   <213> Artificial Sequence
<220>
   <223> Description of Artificial Sequence:oligonucleotide primer for PCR amplification of Ra12 C-terminal fragment of MTB32A
<400> 13
   caattacata tgcatcacca tcaccatcac acggccgcgt ccgataactt c 51
<210> 14
   <211> 33
   <212> DNA
   <213> Artificial Sequence
<220>
   <223> Description of Artificial Sequence:3' oligonucleotide primer for PCR amplification of Ra12 C-terminal fragment of MTB32A
<400> 14
   ctaatcgaat tcggccgggg gtccctcggc caa 33
<210> 15
   <211> 48
   <212> DNA
   <213> Artificial Sequence
<220>
   <223> Description of Artificial Sequence:5' oligonucleotide primer containing enterokinase recognition site for PCR amplification of DPPD mature secreted form
<400> 15
   caattagaat tcgacgacga cgacaaggat ccacctgacc cgcatcag 48
<210> 16
   <211> 33
   <212> DNA
   <213> Artificial Sequence
<220>
   <223> Description of Artificial Sequence:3' oligonucleotide primer containing enterokinase recognition site for PCR amplification of DPPD mature secreted form
<400> 16
   caattagaat tctcagggag cgttgggctg ctc 33
<210> 17
   <211> 30
   <212> PRT
   <213> Artificial Sequence
<220>
   <223> Description of Artificial Sequence:Ra12(short) polypeptide
<400> 17
   Thr Ala Ala Ser Asp Asn Phe Gln Leu Ser Gln Gly Gly Gln Gly Phe
    1               5                  10                  15
   Ala Ile Pro Ile Gly Gln Ala Met Ala Ile Ala Gly Gln Ile
               20                  25                  30
<210> 18
   <211> 128
   <212> PRT
   <213> Artificial Sequence
<220>
   <223> Description of Artificial Sequence:Ra12(long) polypeptide
<400> 18
   Thr Ala Ala Ser Asp Asn Phe Gln Leu Ser Gln Gly Gly Gln Gly Phe
    1               5                  10                  15
   Ala Ile Pro Ile Gly Gln Ala Met Ala Ile Ala Gly Gln Ile Lys Leu
               20                  25                  30
   Pro Thr Val His Ile Gly Pro Thr Ala Phe Leu Gly Leu Gly Val Val
           35                  40                  45
   Asp Asn Asn Gly Asn Gly Ala Arg Val Gln Arg Val Val Gly Ser Ala
       50                  55                  60
   Pro Ala Ala Ser Leu Gly Ile Ser Thr Gly Asp Val Ile Thr Ala Val
   65                  70                  75                  80
   Asp Gly Ala Pro Ile Asn Ser Ala Thr Ala Met Ala Asp Ala Leu Asn
                   85                  90                  95
   Gly His His Pro Gly Asp Val Ile Ser Val Thr Trp Gln Thr Lys Ser
               100                 105                 110
   Gly Gly Thr Arg Thr Gly Asn Val Thr Leu Ala Glu Gly Pro Pro Ala
           115                 120                 125
<210> 19
   <211> 34
   <212> DNA
   <213> Artificial Sequence
<220>
   <223> Description of Artificial Sequence:5' oligonucleotide primer, HindIII site, for PCR amplification of human mammaglobin
<400> 19
   gcgaagctta tgaagttgct gatggtcctc atgc 34
<210> 20
   <211> 36
   <212> DNA
   <213> Artificial Sequence
<220>
   <223> Description of Artificial Sequence:3' oligonucleotide primer, XhoI site, for PCR amplification of human mammaglobin
<400> 20
   cggctcgagt taaaataaat cacaaagact gctgtc 36
<210> 21
   <211> 7
   <212> PRT
   <213> Artificial Sequence
<220>
   <223> Description of Artificial Sequence:Met-His tag 6aa
<400> 21
   Met His His His His His His
   1               5
<210> 22
   <211> 4
   <212> PRT
   <213> Artificial Sequence
<220>
   <223> Description of Artificial Sequence:enterokinase recognition site
<400> 22
   Asp Asp Asp Lys
     1

### SEQUENCE LISTING

<110> Corixa Corporation
<120> Methods of Using a Mycobacterium Tuberculosis Coding Sequence to Facilitate Stable and High Yield Expression of Heterologous Proteins
<130> AHB/FP6050272
<140> EP 00970619.3
   <141> 2000-10-06
<150> PCT/US00/27652
   <151> 2060-10-06
<150> US 60/158,585
   <151> 1999-10-07
<160> 22
<170> PatentIn Ver. 2.1
<210> 1
   <211> 1872
   <212> DNA
   <213> Mycobacterium tuberculosis
<220>
   <223> 32 KD serine protease MTB32A
<220>
   <221> CDS
   <222> (89)..(1156)
   <223> MTB32A
<220>
   <221> sig_peptide
   <222> (89)..(184)
   <223> N-terminal hydrophobic secretory signal sequence
<220>
   <221> mat_peptide
   <222> (185)..(1153)
<400> 1
   gactacgttg gtgtagaaaa atcctgccgc ccggaccctt aaggctggga caatttctga 60
   tagctacccc gacacaggag gttacggg atg agc aat tcg cgc cgc cgc tca     112
                                  Met Ser Asn Ser Arg Arg Arg Ser
                                          -30                 -25
   ctc agg tgg tca tgg ttg ctg agc gtg ctg gct gcc gtc ggg ctg ggc   160
   Leu Arg Trp Ser Trp Leu Leu Ser Val Leu Ala Ala Val Gly Leu Gly
                   -20                 -15                 -10
   ctg gcc acg gcg ccg gcc cag gcg gcc ccg ccg gcc ttg tcg cag gac   208
   Leu Ala Thr Ala Pro Ala Gln Ala Ala Pro Pro Ala Leu Ser Gln Asp
                -5                  -1   1               5
   cgg ttc gcc gac ttc ccc gcg ctg ccc ctc gac ccg tcc gcg atg gtc   256
   Arg Phe Ala Asp Phe Pro Ala Leu Pro Leu Asp Pro Ser Ala Met Val
         10                  15                  20
   gcc caa gtg ggg cca cag gtg gtc aac atc aac acc aaa ctg ggc tac      304
   Ala Gln Val Gly Pro Gln Val Val Asn Ile Asn Thr Lys Leu Gly Tyr
    25                  30                  35                  40
   aac aac gcc gtg ggc gcc ggg acc ggc atc gtc atc gat ccc aac ggt      352
   Asn Asn Ala Val Gly Ala Gly Thr Gly Ile Val Ile Asp Pro Asn Gly
                    45                  50                  55
   gtc gtg ctg acc aac aac cac gtg atc gcg ggc gcc acc gac atc aat      400
   Val Val Leu Thr Asn Asn His Val Ile Ala Gly Ala Thr Asp Ile Asn
                60                  65                  70
   gcg ttc agc gtc ggc tcc ggc caa acc tac ggc gtc gat gtg gtc ggg      448
   Ala Phe Ser Val Gly Ser Gly Gln Thr Tyr Gly Val Asp Val Val Gly
            75                  80                  85
   tat gac cgc acc cag gat gtc gcg gtg ctg cag ctg cgc ggt gcc ggt      496
   Tyr Asp Arg Thr Gln Asp Val Ala Val Leu Gln Leu Arg Gly Ala Gly
        90                  95                 100
   ggc ctg ccg tcg gcg gcg atc ggt ggc ggc gtc gcg gtt ggt gag ccc      544
   Gly Leu Pro Ser Ala Ala Ile Gly Gly Gly Val Ala Val Gly Glu Pro
   105                 110                 115                 120
   gtc gtc gcg atg ggc aac agc ggt ggg cag ggc gga acg ccc cgt gcg      592
   Val Val Ala Met Gly Asn Ser Gly Gly Gln Gly Gly Thr Pro Arg Ala
                   125                 130                 135
   gtg cct ggc agg gtg gtc gcg ctc ggc caa acc gtg cag gcg tcg gat      640
   Val Pro Gly Arg Val Val Ala Leu Gly Gln Thr Val Gln Ala Ser Asp
               140                 145                 150
   tcg ctg acc ggt gcc gaa gag aca ttg aac ggg ttg atc cag ttc gat      688
   Ser Leu Thr Gly Ala Glu Glu Thr Leu Asn Gly Leu Ile Gln Phe Asp
           155                 160                 165
   gcc gcg atc cag ccc ggt gat tcg ggc ggg ccc gtc gtc aac ggc cta      736
   Ala Ala Ile Gln Pro Gly Asp Ser Gly Gly Pro Val Val Asn Gly Leu
       170                 175                 180
   gga cag gtg gtc ggt atg aac acg gcc gcg tcc gat aac ttc cag ctg      784
   Gly Gln Val Val Gly Met Asn Thr Ala Ala Ser Asp Asn Phe Gln Leu
   185                 190                 195                 200
   tcc cag ggt ggg cag gga ttc gcc att ccg atc ggg cag gcg atg gcg      832
   Ser Gln Gly Gly Gln Gly Phe Ala Ile Pro Ile Gly Gln Ala Met Ala
                   205                 210                 215
   atc gcg ggc cag atc cga tcg ggt ggg ggg tca ccc acc gtt cat atc      880
   Ile Ala Gly Gln Ile Arg Ser Gly Gly Gly Ser Pro Thr Val His Ile
               220                 225                 230
   ggg cct acc gcc ttc ctc ggc ttg ggt gtt gtc gac aac aac ggc aac      928
   Gly Pro Thr Ala Phe Leu Gly Leu Gly Val Val Asp Asn Asn Gly Asn
           235                 240                 245
   ggc gca cga gtc caa cgc gtg gtc ggg agc gct ccg gcg gca agt ctc      976
   Gly Ala Arg Val Gln Arg Val Val Gly Ser Ala Pro Ala Ala Ser Leu
       250                 255                 260
   ggc atc tcc acc ggc gac gtg atc acc gcg gtc gac ggc gct ccg atc    1024
   Gly Ile Ser Thr Gly Asp Val Ile Thr Ala Val Asp Gly Ala Pro Ile
   265                 270                 275                 280
   aac tcg gcc acc gcg atg gcg gac gcg ctt aac ggg cat cat ccc ggt    1072
   Asn Ser Ala Thr Ala Met Ala Asp Ala Leu Asn Gly His His Pro Gly
                   285                 290                 295
   gac gtc atc tcg gtg acc tgg caa acc aag tcg ggc ggc acg cgt aca    1120
   Asp Val Ile Ser Val Thr Trp Gln Thr Lys Ser Gly Gly Thr Arg Thr
               300                 305                 310
   ggg aac gtg aca ttg gcc gag gga ccc ccg gcc tga tttcgtcgcg         1166
   Gly Asn Val Thr Leu Ala Glu Gly Pro Pro Ala
           315                 320
   gataccaccc gccggccggc caattggatt ggcgccagcc gtgattgccg cgtgagcccc 1226
   cgagttccgt ctcccgtgcg cgtggcatcg tggaagcaat gaacgaggca gaacacagcg 1286
   tcgagcaccc tcccgtgcag ggcagtcacg tcgaaggcgg tgtggtcgag catccggatg 1346
   ccaaggactt cggcagcgcc gccgccctgc ccgccgatcc gacctggttt aagcacgccg 1406
   tcttctacga ggtgctggtc cgggcgttct tcgacgccag cgcggacggt tccggcgatc 1466
   tgcgtggact catcgatcgc ctcgactacc tgcagtggct tggcatcgac tgcatctggt 1526
   tgccgccgtt ctacgactcg ccgctgcgcg acggcggtta cgacattcgc gacttctaca 1586
   aggtgctgcc cgaattcggc accgtcgacg atttcgtcgc cctggtcgac gccgctcacc 1646
   ggcgaggtat ccgcatcatc accgacctgg tgatgaatca cacctcggag tcgcacccct 1706
   ggtttcagga gtcccgccgc gacccagacg gaccgtacgg tgactattac gtgtggagcg 1766
   acaccagcga gcgctacacc gacgcccgga tcatcttcgt cgacaccgaa gagtcgaact 1826
   ggtcattcga tcctgtccgc cgacagttct actggcaccg attctt                1872
<210> 2
   <211> 355
   <212> PRT
   <213> Mycobacterium tuberculosis
   <223> 32 KD serine protease MTB32A
<400> 2
   Met Ser Asn Ser Arg Arg Arg Ser Leu Arg Trp Ser Trp Leu Leu Ser
     1               5                  10                  15
   Val Leu Ala Ala Val Gly Leu Gly Leu Ala Thr Ala Pro Ala Gln Ala
                20                  25                  30
   Ala Pro Pro Ala Leu Ser Gln Asp Arg Phe Ala Asp Phe Pro Ala Leu
            35                  40                  45
   Pro Leu Asp Pro Ser Ala Met Val Ala Gln Val Gly Pro Gln Val Val
        50                  55                  60
   Asn Ile Asn Thr Lys Leu Gly Tyr Asn Asn Ala Val Gly Ala Gly Thr
    65                  70                  75                  80
   Gly Ile Val Ile Asp Pro Asn Gly Val Val Leu Thr Asn Asn His Val
                    85                  90                  95
   Ile Ala Gly Ala Thr Asp Ile Asn Ala Phe Ser Val Gly Ser Gly Gln
               100                 105                 110
   Thr Tyr Gly Val Asp Val Val Gly Tyr Asp Arg Thr Gln Asp Val Ala
           115                 120                 125
   Val Leu Gln Leu Arg Gly Ala Gly Gly Leu Pro Ser Ala Ala Ile Gly
       130                 135                 140
   Gly Gly Val Ala Val Gly Glu Pro Val Val Ala Met Gly Asn Ser Gly
   145                 150                 155                 160
   Gly Gln Gly Gly Thr Pro Arg Ala Val Pro Gly Arg Val Val Ala Leu
                   165                 170                 175
   Gly Gln Thr Val Gln Ala Ser Asp Ser Leu Thr Gly Ala Glu Glu Thr
               180                 185                 190
   Leu Asn Gly Leu Ile Gln Phe Asp Ala Ala Ile Gln Pro Gly Asp Ser
           195                 200                 205
   Gly Gly Pro Val Val Asn Gly Leu Gly Gln Val Val Gly Met Asn Thr
       210                 215                 220
   Ala Ala Ser Asp Asn Phe Gln Leu Ser Gln Gly Gly Gln Gly Phe Ala
   225                 230                 235                 240
   Ile Pro Ile Gly Gln Ala Met Ala Ile Ala Gly Gln Ile Arg Ser Gly
                   245                 250                 255
   Gly Gly Ser Pro Thr Val His Ile Gly Pro Thr Ala Phe Leu Gly Leu
               260                 265                 270
   Gly Val Val Asp Asn Asn Gly Asn Gly Ala Arg Val Gln Arg Val Val
           275                 280                 285
   Gly Ser Ala Pro Ala Ala Ser Leu Gly Ile Ser Thr Gly Asp Val Ile
       290                 295                 300
   Thr Ala Val Asp Gly Ala Pro Ile Asn Ser Ala Thr Ala Met Ala Asp
   305                 310                 315                 320
   Ala Leu Asn Gly His His Pro Gly Asp Val Ile Ser Val Thr Trp Gln
                   325                 330                 335
   Thr Lys Ser Gly Gly Thr Arg Thr Gly Asn Val Thr Leu Ala Glu Gly
               340                 345                 350
   Pro Pro Ala
           355
<210> 3
   <211> 396
   <212> DNA
   <213> Mycobacterium tuberculosis
<220>
   <223> 14 KD C-terminal fragment of MTB32A Ra12
<220>
   <221> CDS
   <222> (1)..(396)
   <223> Ra12
<400> 3
   acg gcc gcg tcc gat aac ttc cag ctg tcc cag ggt ggg cag gga ttc    48
   Thr Ala Ala Ser Asp Asn Phe Gln Leu Ser Gln Gly Gly Gln Gly Phe
   1               5                   10                  15
   gcc att ccg atc ggg cag gcg atg gcg atc gcg ggc cag atc cga tcg    96
   Ala Ile Pro Ile Gly Gln Ala Met Ala Ile Ala Gly Gln Ile Arg Ser
               20                  25                  30
   ggt ggg ggg tca ccc acc gtt cat atc ggg cct acc gcc ttc ctc ggc    144
   Gly Gly Gly Ser Pro Thr Val His Ile Gly Pro Thr Ala Phe Leu Gly
           35                  40                  45
   ttg ggt gtt gtc gac aac aac ggc aac ggc gca cga gtc caa cgc gtg    192
   Leu Gly Val Val Asp Asn Asn Gly Asn Gly Ala Arg Val Gln Arg Val
       50                  55                  60
   gtc ggg agc gct ccg gcg gca agt ctc ggc atc tcc acc ggc gac gtg    240
   Val Gly Ser Ala Pro Ala Ala Ser Leu Gly Ile Ser Thr Gly Asp Val
   65                  70                  75                  80
   atc acc gcg gtc gac ggc gct ccg atc aac tcg gcc acc gcg atg gcg    288
   Ile Thr Ala Val Asp Gly Ala Pro Ile Asn Ser Ala Thr Ala Met Ala
                   85                  90                  95
   gac gcg ctt aac ggg cat cat ccc ggt gac gtc atc tcg gtg acc tgg    336
   Asp Ala Leu Asn Gly His His Pro Gly Asp Val Ile Ser Val Thr Trp
               100                 105                 110
   caa acc aag tcg ggc ggc acg cgt aca ggg aac gtg aca ttg gcc gag    384
   Gln Thr Lys Ser Gly Gly Thr Arg Thr Gly Asn Val Thr Leu Ala Glu
           115                 120                 125
   gga ccc ccg gcc                                                    396
   Gly Pro Pro Ala
       130
<210> 4
   <211> 132
   <212> PRT
   <213> Mycobacterium tuberculosis
   <223> 14 KD C-terminal fragment of MTB32A Ra12
<400> 4
   Thr Ala Ala Ser Asp Asn Phe Gln Leu Ser Gln Gly Gly Gln Gly Phe
   1               5                   10                  15
   Ala Ile Pro Ile Gly Gln Ala Met Ala Ile Ala Gly Gln Ile Arg Ser
               20                  25                  30
   Gly Gly Gly Ser Pro Thr Val His Ile Gly Pro Thr Ala Phe Leu Gly
           35                  40                  45
   Leu Gly Val Val Asp Asn Asn Gly Asn Gly Ala Arg Val Gln Arg Val
       50                  55                  60
   Val Gly Ser Ala Pro Ala Ala Ser Leu Gly Ile Ser Thr Gly Asp Val
   65                  70                  75                  80
   Ile Thr Ala Val Asp Gly Ala Pro Ile Asn Ser Ala Thr Ala Met Ala
                   85                  90                  95
   Asp Ala Leu Asn Gly His His Pro Gly Asp Val Ile Ser Val Thr Trp
               100                 105                 110
   Gln Thr Lys Ser Gly Gly Thr Arg Thr Gly Asn Val Thr Leu Ala Glu
           115                 120                 125
   Gly Pro Pro Ala
       130
<210> 5
   <211> 702
   <212> DNA
   <213> Artificial Sequence
<220>
   <221> CDS
   <222> (4)..(696)
   <223> Ra12-DPPD fusion polypeptide
<220>
   <223> Description of Artificial Sequence:Ra12-DPPD fusion polypeptide
<400> 5
   cat atg cat cac cat cac cat cac acg gcc gcg tcc gat aac ttc cag    48
       Met His His His His His His Thr Ala Ala Ser Asp Asn Phe Gln
         1               5                  10                  15
   ctg tcc cag ggt ggg cag gga ttc gcc att ccg atc ggg cag gcg atg    96
   Leu Ser Gln Gly Gly Gln Gly Phe Ala Ile Pro Ile Gly Gln Ala Met
                    20                  25                  30
   gcg atc gcg ggc cag atc cga tcg ggt ggg ggg tca ccc acc gtt cat   144
   Ala Ile Ala Gly Gln Ile Arg Ser Gly Gly Gly Ser Pro Thr Val His
                35                  40                  45
   atc ggg cct acc gcc ttc ctc ggc ttg ggt gtt gtc gac aac aac ggc   192
   Ile Gly Pro Thr Ala Phe Leu Gly Leu Gly Val Val Asp Asn Asn Gly
            50                  55                  60
   aac ggc gca cga gtc caa cgc gtg gtc ggg agc gct ccg gcg gca agt   240
   Asn Gly Ala Arg Val Gln Arg Val Val Gly Ser Ala Pro Ala Ala Ser
        65                  70                  75
   ctc ggc atc tcc acc ggc gac gtg atc acc gcg gtc gac ggc gct ccg   288
   Leu Gly Ile Ser Thr Gly Asp Val Ile Thr Ala Val Asp Gly Ala Pro
    80                  85                  90                  95
   atc aac tcg gcc acc gcg atg gcg gac gcg ctt aac ggg cat cat ccc   336
   Ile Asn Ser Ala Thr Ala Met Ala Asp Ala Leu Asn Gly His His Pro
                   100                 105                 110
   ggt gac gtc atc tcg gtg acc tgg caa acc aag tcg ggc ggc acg cgt   384
   Gly Asp Val Ile Ser Val Thr Trp Gln Thr Lys Ser Gly Gly Thr Arg
               115                 120                 125
   aca ggg aac gtg aca ttg gcc gag gga ccc ccg gcc gaa ttc gac gac   432
   Thr Gly Asn Val Thr Leu Ala Glu Gly Pro Pro Ala Glu Phe Asp Asp
           130                 135                 140
   gac gac aag gat cca cct gac ccg cat cag ccg gac atg acg aaa ggc   480
   Asp Asp Lys Asp Pro Pro Asp Pro His Gln Pro Asp Met Thr Lys Gly
       145                 150                 155
   tat tgc ccg ggt ggc cga tgg ggt ttt ggc gac ttg gcc gtg tgc gac   528
   Tyr Cys Pro Gly Gly Arg Trp Gly Phe Gly Asp Leu Ala Val Cys Asp
   160                 165                 170                 175
   ggc gag aag tac ccc gac ggc tcg ttt tgg cac cag tgg atg caa acg   576
   Gly Glu Lys Tyr Pro Asp Gly Ser Phe Trp His Gln Trp Met Gln Thr
                   180                 185                 190
   tgg ttt acc ggc cca cag ttt tac ttc gat tgt gtc agc ggc ggt gag   624
   Trp Phe Thr Gly Pro Gln Phe Tyr Phe Asp Cys Val Ser Gly Gly Glu
               195                 200                 205
   ccc ctc ccc ggc ccg ccg cca ccg ggt ggt tgc ggt ggg gca att ccg   672
   Pro Leu Pro Gly Pro Pro Pro Pro Gly Gly Cys Gly Gly Ala Ile Pro
           210                 215                 220
   tcc gag cag ccc aac gct ccc tga gaattc                                   702
   Ser Glu Gln Pro Asn Ala Pro
       225                 230
<210> 6
   <211> 230
   <212> PRT
   <213> Artificial Sequence
   <223> Description of Artificial Sequence:Ra12-DPPD fusion polypeptide
<400> 6
   Met His His His His His His Thr Ala Ala Ser Asp Asn Phe Gln Leu
     1               5                  10                  15
   Ser Gln Gly Gly Gln Gly Phe Ala Ile Pro Ile Gly Gln Ala Met Ala
               20                  25                  30
   Ile Ala Gly Gln Ile Arg Ser Gly Gly Gly Ser Pro Thr Val His Ile
           35                  40                  45
   Gly Pro Thr Ala Phe Leu Gly Leu Gly Val Val Asp Asn Asn Gly Asn
       50                  55                  60
   Gly Ala Arg Val Gln Arg Val Val Gly Ser Ala Pro Ala Ala Ser Leu
    65                  70                  75                  80
   Gly Ile Ser Thr Gly Asp Val Ile Thr Ala Val Asp Gly Ala Pro Ile
                   85                  90                  95
   Asn Ser Ala Thr Ala Met Ala Asp Ala Leu Asn Gly His His Pro Gly
               100                 105                 110
   Asp Val Ile Ser Val Thr Trp Gln Thr Lys Ser Gly Gly Thr Arg Thr
           115                 120                 125
   Gly Asn Val Thr Leu Ala Glu Gly Pro Pro Ala Glu Phe Asp Asp Asp
       130                 135                 140
   Asp Lys Asp Pro Pro Asp Pro His Gln Pro Asp Met Thr Lys Gly Tyr
   145                 150                 155                 160
   Cys Pro Gly Gly Arg Trp Gly Phe Gly Asp Leu Ala Val Cys Asp Gly
                   165                 170                 175
   Glu Lys Tyr Pro Asp Gly Ser Phe Trp His Gln Trp Met Gln Thr Trp
               180                 185                 190
   Phe Thr Gly Pro Gln Phe Tyr Phe Asp Cys Val Ser Gly Gly Glu Pro
           195                 200                 205
   Leu Pro Gly Pro Pro Pro Pro Gly Gly Cys Gly Gly Ala Ile Pro Ser
       210                 215                 220
   Glu Gln Pro Asn Ala Pro
   225                 230
<210> 7
   <211> 1746
   <212> DNA
   <213> Artificial Sequence
<220>
   <223> Description of Artificial Sequence:Ra12-WT1 fusion
<220>
   <221> CDS
   <222> (4) .. (1740)
   <223> Ra12-WT1 fusion polypeptide
<400> 7
   cat atg cat cac cat cac cat cac acg gcc gcg tcc gat aac ttc cag    48
       Met His His His His His His Thr Ala Ala Ser Asp Asn Phe Gln
        1               5                  10                  15
   ctg tcc cag ggt ggg cag gga ttc gcc att ccg atc ggg cag gcg atg    96
   Leu Ser Gln Gly Gly Gln Gly Phe Ala Ile Pro Ile Gly Gln Ala Met
                   20                  25                  30
   gcg atc gcg ggc cag atc cga tcg ggt ggg ggg tca ccc acc gtt cat   144
   Ala Ile Ala Gly Gln Ile Arg Ser Gly Gly Gly Ser Pro Thr Val His
               35                  40                  45
   atc ggg cct acc gcc ttc ctc ggc ttg ggt gtt gtc gac aac aac ggc   192
   Ile Gly Pro Thr Ala Phe Leu Gly Leu Gly Val Val Asp Asn Asn Gly
           50                  55                  60
   aac ggc gca cga gtc caa cgc gtg gtc ggg agc gct ccg gcg gca agt   240
   Asn Gly Ala Arg Val Gln Arg Val Val Gly Ser Ala Pro Ala Ala Ser
       65                  70                  75
   ctc ggc atc tcc acc ggc gac gtg atc acc gcg gtc gac ggc gct ccg   288
   Leu Gly Ile Ser Thr Gly Asp Val Ile Thr Ala Val Asp Gly Ala Pro
   80                  85                  90                  95
   atc aac tcg gcc acc gcg atg gcg gac gcg ctt aac ggg cat cat ccc   336
   Ile Asn Ser Ala Thr Ala Met Ala Asp Ala Leu Asn Gly His His Pro
                   100                 105                 110
   ggt gac gtc atc tcg gtg acc tgg caa acc aag tcg ggc ggc acg cgt   384
   Gly Asp Val Ile Ser Val Thr Trp Gln Thr Lys Ser Gly Gly Thr Arg
               115                 120                 125
   aca ggg aac gtg aca ttg gcc gag gga ccc ccg gcc gaa ttc ccg ctg   432
   Thr Gly Asn Val Thr Leu Ala Glu Gly Pro Pro Ala Glu Phe Pro Leu
           130                 135                 140
   gtg ccg cgc ggc agc ccg atg ggc tcc gac gtt cgg gac ctg aac gca   480
   Val Pro Arg Gly Ser Pro Met Gly Ser Asp Val Arg Asp Leu Asn Ala
       145                 150                 155
   ctg ctg ccg gca gtt ccg tcc ctg ggt ggt ggt ggt ggt tgc gca ctg   528
   Leu Leu Pro Ala Val Pro Ser Leu Gly Gly Gly Gly Gly Cys Ala Leu
   160                 165                 170                 175
   ccg gtt agc ggt gca gca cag tgg gct ccg gtt ctg gac ttc gca ccg   576
   Pro Val Ser Gly Ala Ala Gln Trp Ala Pro Val Leu Asp Phe Ala Pro
                   180                 185                 190
   ccg ggt gca tcc gca tac ggt tcc ctg ggt ggt ccg gca ccg ccg ccg   624
   Pro Gly Ala Ser Ala Tyr Gly Ser Leu Gly Gly Pro Ala Pro Pro Pro
               195                 200                 205
   gca ccg ccg ccg ccg ccg ccg ccg ccg ccg cac tcc ttc atc aaa cag   672
   Ala Pro Pro Pro Pro Pro Pro Pro Pro Pro His Ser Phe Ile Lys Gln
           210                 215                 220
   gaa ccg agc tgg ggt ggt gca gaa ccg cac gaa gaa cag tgc ctg agc   720
   Glu Pro Ser Trp Gly Gly Ala Glu Pro His Glu Glu Gln Cys Leu Ser
       225                 230                 235
   gca ttc acc gtt cac ttc tcc ggc cag ttc act ggc aca gcc gga gcc   768
   Ala Phe Thr Val His Phe Ser Gly Gln Phe Thr Gly Thr Ala Gly Ala
   240                 245                 250                 255
   tgt cgc tac ggg ccc ttc ggt cct cct ccg ccc agc cag gcg tca tcc     816
   Cys Arg Tyr Gly Pro Phe Gly Pro Pro Pro Pro Ser Gln Ala Ser Ser
                   260                 265                 270
   ggc cag gcc agg atg ttt cct aac gcg ccc tac ctg ccc agc tgc ctc     864
   Gly Gln Ala Arg Met Phe Pro Asn Ala Pro Tyr Leu Pro Ser Cys Leu
               275                 280                 285
   gag agc cag ccc gct att cgc aat cag ggt tac agc acg gtc acc ttc     912
   Glu Ser Gln Pro Ala Ile Arg Asn Gln Gly Tyr Ser Thr Val Thr Phe
           290                 295                 300
   gac ggg acg ccc agc tac ggt cac acg ccc tcg cac cat gcg gcg cag     960
   Asp Gly Thr Pro Ser Tyr Gly His Thr Pro Ser His His Ala Ala Gln
       305                 310                 315
   ttc ccc aac cac tca ttc aag cat gag gat ccc atg ggc cag cag ggc    1008
   Phe Pro Asn His Ser Phe Lys His Glu Asp Pro Met Gly Gln Gln Gly
   320                 325                 330                 335
   tcg ctg ggt gag cag cag tac tcg gtg ccg ccc ccg gtc tat ggc tgc    1056
   Ser Leu Gly Glu Gln Gln Tyr Ser Val Pro Pro Pro Val Tyr Gly Cys
                   340                 345                 350
   cac acc ccc acc gac agc tgc acc ggc agc cag gct ttg ctg ctg agg    1104
   His Thr Pro Thr Asp Ser Cys Thr Gly Ser Gln Ala Leu Leu Leu Arg
               355                 360                 365
   acg ccc tac agc agt gac aat tta tac caa atg aca tcc cag ctt gaa    1152
   Thr Pro Tyr Ser Ser Asp Asn Leu Tyr Gln Met Thr Ser Gln Leu Glu
           370                 375                 380
   tgc atg acc tgg aat cag atg aac tta gga gcc acc tta aag ggc cac    1200
   Cys Met Thr Trp Asn Gln Met Asn Leu Gly Ala Thr Leu Lys Gly His
       385                 390                 395
   agc aca ggg tac gag agc gat aac cac aca acg ccc atc ctc tgc gga    1248
   Ser Thr Gly Tyr Glu Ser Asp Asn His Thr Thr Pro Ile Leu Cys Gly
   400                 405                 410                 415
   gcc caa tac aga ata cac acg cac ggt gtc ttc aga ggc att cag gat    1296
   Ala Gln Tyr Arg Ile His Thr His Gly Val Phe Arg Gly Ile Gln Asp
                   420                 425                 430
   gtg cga cgt gtg cct gga gta gcc ccg act ctt gta cgg tcg gca tct    1344
   Val Arg Arg Val Pro Gly Val Ala Pro Thr Leu Val Arg Ser Ala Ser
               435                 440                 445
   gag acc agt gag aaa cgc ccc ttc atg tgt gct tac tca ggc tgc aat    1392
   Glu Thr Ser Glu Lys Arg Pro Phe Met Cys Ala Tyr Ser Gly Cys Asn
           450                 455                 460
   aag aga tat ttt aag ctg tcc cac tta cag atg cac agc agg aag cac    1440
   Lys Arg Tyr Phe Lys Leu Ser His Leu Gln Met His Ser Arg Lys His
       465                 470                 475
   act ggt gag aaa cca tac cag tgt gac ttc aag gac tgt gaa cga agg    1488
   Thr Gly Glu Lys Pro Tyr Gln Cys Asp Phe Lys Asp Cys Glu Arg Arg
   480                 485                 490                 495
   ttt ttt cgt tca gac cag ctc aaa aga cac caa agg aga cat aca ggt    1536
   Phe Phe Arg Ser Asp Gln Leu Lys Arg His Gln Arg Arg His Thr Gly
                   500                 505                 510
   gtg aaa cca ttc cag tgt aaa act tgt cag cga aag ttc tcc cgg tcc    1584
   Val Lys Pro Phe Gln Cys Lys Thr Cys Gln Arg Lys Phe Ser Arg Ser
               515                 520                 525
   gac cac ctg aag acc cac acc agg act cat aca ggt gaa aag ccc ttc    1632
   Asp His Leu Lys Thr His Thr Arg Thr His Thr Gly Glu Lys Pro Phe
           530                 535                 540
   agc tgt cgg tgg cca agt tgt cag aaa aag ttt gcc cgg tca gat gaa    1680
   Ser Cys Arg Trp Pro Ser Cys Gln Lys Lys Phe Ala Arg Ser Asp Glu
       545                 550                 555
   tta gtc cgc cat cac aac atg cat cag aga aac atg acc aaa ctc cag    1728
   Leu Val Arg His His Asn Met His Gln Arg Asn Met Thr Lys Leu Gln
   560                 565                 570                 575
   ctg gcg ctt tga gaattc                                             1746
   Leu Ala Leu
<210> 8
   <211> 578
   <212> PRT
   <213> Artificial Sequence <223> Description of Artificial Sequence:Ra12-WT1 fusion polypeptide
<400> 8
   Met His His His His His His Thr Ala Ala Ser Asp Asn Phe Gln Leu
    1               5                  10                  15
   Ser Gln Gly Gly Gln Gly Phe Ala Ile Pro Ile Gly Gln Ala Met Ala
               20                  25                  30
   Ile Ala Gly Gln Ile Arg Ser Gly Gly Gly Ser Pro Thr Val His Ile
           35                  40                  45
   Gly Pro Thr Ala Phe Leu Gly Leu Gly Val Val Asp Asn Asn Gly Asn
       50                  55                  60
   Gly Ala Arg Val Gln Arg Val Val Gly Ser Ala Pro Ala Ala Ser Leu
   65                  70                  75                  80
   Gly Ile Ser Thr Gly Asp Val Ile Thr Ala Val Asp Gly Ala Pro Ile
                   85                  90                  95
   Asn Ser Ala Thr Ala Met Ala Asp Ala Leu Asn Gly His His Pro Gly
               100                 105                 110
   Asp Val Ile Ser Val Thr Trp Gln Thr Lys Ser Gly Gly Thr Arg Thr
           115                 120                 125
   Gly Asn Val Thr Leu Ala Glu Gly Pro Pro Ala Glu Phe Pro Leu Val
       130                 135                 140
   Pro Arg Gly Ser Pro Met Gly Ser Asp Val Arg Asp Leu Asn Ala Leu
   145                 150                 155                 160
   Leu Pro Ala Val Pro Ser Leu Gly Gly Gly Gly Gly Cys Ala Leu Pro
                   165                 170                 175
   Val Ser Gly Ala Ala Gln Trp Ala Pro Val Leu Asp Phe Ala Pro Pro
               180                 185                 190
   Gly Ala Ser Ala Tyr Gly Ser Leu Gly Gly Pro Ala Pro Pro Pro Ala
           195                 200                 205
   Pro Pro Pro Pro Pro Pro Pro Pro Pro His Ser Phe Ile Lys Gln Glu
           210                     215                     220
   Pro Ser Trp Gly Gly Ala Glu Pro His Glu Glu Gln Cys Leu Ser Ala
   225                 230                     235                 240
   Phe Thr Val His Phe Ser Gly Gln Phe Thr Gly Thr Ala Gly Ala Cys
                   245                     250                     255
   Arg Tyr Gly Pro Phe Gly Pro Pro Pro Pro Ser Gln Ala Ser Ser Gly
               260                     265                     270
   Gln Ala Arg Met Phe Pro Asn Ala Pro Tyr Leu Pro Ser Cys Leu Glu
           275                     280                     285
   Ser Gln Pro Ala Ile Arg Asn Gln Gly Tyr Ser Thr Val Thr Phe Asp
       290                     295                     300
   Gly Thr Pro Ser Tyr Gly His Thr Pro Ser His His Ala Ala Gln Phe
   305                 310                     315                 320
   Pro Asn His Ser Phe Lys His Glu Asp Pro Met Gly Gln Gln Gly Ser
                   325                     330                     335
   Leu Gly Glu Gln Gln Tyr Ser Val Pro Pro Pro Val Tyr Gly Cys His
               340                     345                     350
   Thr Pro Thr Asp Ser Cys Thr Gly Ser Gln Ala Leu Leu Leu Arg Thr
           355                     360                     365
   Pro Tyr Ser Ser Asp Asn Leu Tyr Gln Met Thr Ser Gln Leu Glu Cys
       370                     375                     380
   Met Thr Trp Asn Gln Met Asn Leu Gly Ala Thr Leu Lys Gly His Ser
   385                 390                     395                 400
   Thr Gly Tyr Glu Ser Asp Asn His Thr Thr Pro Ile Leu Cys Gly Ala
                   405                     410                     415
   Gln Tyr Arg Ile His Thr His Gly Val Phe Arg Gly Ile Gln Asp Val
               420                     425                     430
   Arg Arg Val Pro Gly Val Ala Pro Thr Leu Val Arg Ser Ala Ser Glu
           435                     440                     445
   Thr Ser Glu Lys Arg Pro Phe Met Cys Ala Tyr Ser Gly Cys Asn Lys
       450                     455                     460
   Arg Tyr Phe Lys Leu Ser His Leu Gln Met His Ser Arg Lys His Thr
   465                 470                     475                 480
   Gly Glu Lys Pro Tyr Gln Cys Asp Phe Lys Asp Cys Glu Arg Arg Phe
                   485                     490                     495
   Phe Arg Ser Asp Gln Leu Lys Arg His Gln Arg Arg His Thr Gly Val
               500                     505                     510
   Lys Pro Phe Gln Cys Lys Thr Cys Gln Arg Lys Phe Ser Arg Ser Asp
           515                     520                     525
   His Leu Lys Thr His Thr Arg Thr His Thr Gly Glu Lys Pro Phe Ser
       530                     535                     540
   Cys Arg Trp Pro Ser Cys Gln Lys Lys Phe Ala Arg Ser Asp Glu Leu
   545                 550                     555                 560
   Val Arg His His Asn Met His Gln Arg Asn Met Thr Lys Leu Gln Leu
                   565                     570                     575
   Ala Leu
<210> 9
   <211> 672
   <212> DNA
   <213> Artificial Sequence
<220>
   <223> Description of Artificial Sequence:Ra12-human mammaglobin fusion
<220>
   <221> CDS
   <222> (4)..(666)
   <223> Ra12-human mammaglobin fusion polypeptide
<400> 9
   cat atg cat cac cat cac cat cac acg gcc gcg tcc gat aac ttc cag     48
   ctg tcc cag ggt ggg cag gga ttc gcc att ccg atc ggg cag gcg atg     96
   gcg atc gcg ggc cag atc cga tcg ggt ggg ggg tca ccc acc gtt cat    144
   atc ggg cct acc gcc ttc ctc ggc ttg ggt gtt gtc gac aac aac ggc    192
   aac ggc gca cga gtc caa cgc gtg gtc ggg agc gct ccg gcg gca agt    240
   ctc ggc atc tcc acc ggc gac gtg atc acc gcg gtc gac ggc gct ccg    288
   atc aac tcg gcc acc gcg atg gcg gac gcg ctt aac ggg cat cat ccc    336
   ggt gac gtc atc tcg gtg acc tgg caa acc aag tcg ggc ggc acg cgt    384
   aca ggg aac gtg aca ttg gcc gag gga ccc ccg gcc gaa ttc atc gag    432
   gga agg ggc tct ggc tgc ccc tta ttg gag aat gtg att tcc aag aca    480
   atc aat cca caa gtg tct aag act gaa tac aaa gaa ctt ctt caa gag    528
   ttc ata gac gac aat gcc act aca aat gcc ata gat gaa ttg aag gaa    576
   tgt ttt ctt aac caa acg gat gaa act ctg agc aat gtt gag gtg ttt    624
   atg caa tta ata tat gac agc agt ctt tgt gat tta ttt taa gaattc     672
<210> 10
   <211> 220
   <212> PRT
   <213> Artificial Sequence
   <223> Description of Artificial Sequence:Ra12-human mammaglobin
   fusion polypeptide
<400> 10
<210> 11
   <211> 2191
   <212> DNA
   <213> Artificial Sequence
<220>
   <223> Description of Artificial Sequence:Ra12-H9-32A fusion (Ra12-MTB39-MTB32A(N-ter) fusion)
<220>
   <221> CDS
   <222> (1)..(2190)
   <223> Ra12-H9-32A (Ra12-MTB39-MTB32A(N-ter)) fusion polypeptide
<400> 11
   atg cat cac cat cac cat cac acg gcc gcg tcc gat aac ttc cag ctg    48
   Met His His His His His His Thr Ala Ala Ser Asp Asn Phe Gln Leu
     1               5                  10                  15
   tcc cag ggt ggg cag gga ttc gcc att ccg atc ggg cag gcg atg gcg    96
   Ser Gln Gly Gly Gln Gly Phe Ala Ile Pro Ile Gly Gln Ala Met Ala
                20                  25                  30
   atc gcg ggc cag atc cga tcg ggt ggg ggg tca ccc acc gtt cat atc   144
   Ile Ala Gly Gln Ile Arg Ser Gly Gly Gly Ser Pro Thr Val His Ile
               35                  40                  45
   ggg cct acc gcc ttc ctc ggc ttg ggt gtt gtc gac aac aac ggc aac      192
   Gly Pro Thr Ala Phe Leu Gly Leu Gly Val Val Asp Asn Asn Gly Asn
       50                  55                  60
   ggc gca cga gtc caa cgc gtg gtc ggg agc gct ccg gcg gca agt ctc      240
   Gly Ala Arg Val Gln Arg Val Val Gly Ser Ala Pro Ala Ala Ser Leu
   65                  70                  75                  80
   ggc atc tcc acc ggc gac gtg atc acc gcg gtc gac ggc gct ccg atc      288
   Gly Ile Ser Thr Gly Asp Val Ile Thr Ala Val Asp Gly Ala Pro Ile
                   85                  90                  95
   aac tcg gcc acc gcg atg gcg gac gcg ctt aac ggg cat cat ccc ggt      336
   Asn Ser Ala Thr Ala Met Ala Asp Ala Leu Asn Gly His His Pro Gly
               100                 105                 110
   gac gtc atc tcg gtg acc tgg caa acc aag tcg ggc ggc acg cgt aca      384
   Asp Val Ile Ser Val Thr Trp Gln Thr Lys Ser Gly Gly Thr Arg Thr
           115                 120                 125
   ggg aac gtg aca ttg gcc gag gga ccc ccg gcc gaa ttc atg gtg gat      432
   Gly Asn Val Thr Leu Ala Glu Gly Pro Pro Ala Glu Phe Met Val Asp
       130                 135                 140
   ttc ggg gcg tta cca ccg gag atc aac tcc gcg agg atg tac gcc ggc      480
   Phe Gly Ala Leu Pro Pro Glu Ile Asn Ser Ala Arg Met Tyr Ala Gly
   145                 150                 155                 160
   ccg ggt tcg gcc tcg ctg gtg gcc gcg gct cag atg tgg gac agc gtg      528
   Pro Gly Ser Ala Ser Leu Val Ala Ala Ala Gln Met Trp Asp Ser Val
                   165                 170                 175
   gcg agt gac ctg ttt tcg gcc gcg tcg gcg ttt cag tcg gtg gtc tgg      576
   Ala Ser Asp Leu Phe Ser Ala Ala Ser Ala Phe Gln Ser Val Val Trp
               180                 185                 190
   ggt ctg acg gtg ggg tcg tgg ata ggt tcg tcg gcg ggt ctg atg gtg      624
   Gly Leu Thr Val Gly Ser Trp Ile Gly Ser Ser Ala Gly Leu Met Val
           195                 200                 205
   gcg gcg gcc tcg ccg tat gtg gcg tgg atg agc gtc acc gcg ggg cag      672
   Ala Ala Ala Ser Pro Tyr Val Ala Trp Met Ser Val Thr Ala Gly Gln
       210                 215                 220
   gcc gag ctg acc gcc gcc cag gtc cgg gtt gct gcg gcg gcc tac gag      720
   Ala Glu Leu Thr Ala Ala Gln Val Arg Val Ala Ala Ala Ala Tyr Glu
   225                 230                 235                 240
   acg gcg tat ggg ctg acg gtg ccc ccg ccg gtg atc gcc gag aac cgt      768
   Thr Ala Tyr Gly Leu Thr Val Pro Pro Pro Val Ile Ala Glu Asn Arg
                   245                 250                 255
   gct gaa ctg atg att ctg ata gcg acc aac ctc ttg ggg caa aac acc      816
   Ala Glu Leu Met Ile Leu Ile Ala Thr Asn Leu Leu Gly Gln Asn Thr
               260                 265                 270
   ccg gcg atc gcg gtc aac gag gcc gaa tac ggc gag atg tgg gcc caa      864
   Pro Ala Ile Ala Val Asn Glu Ala Glu Tyr Gly Glu Met Trp Ala Gln
           275                 280                 285
   gac gcc gcc gcg atg ttt ggc tac gcc gcg gcg acg gcg acg gcg acg    912
   Asp Ala Ala Ala Met Phe Gly Tyr Ala Ala Ala Thr Ala Thr Ala Thr
       290                 295                 300
   gcg acg ttg ctg ccg ttc gag gag gcg ccg gag atg acc agc gcg ggt    960
   Ala Thr Leu Leu Pro Phe Glu Glu Ala Pro Glu Met Thr Ser Ala Gly
   305                 310                 315                 320
   ggg ctc ctc gag cag gcc gcc gcg gtc gag gag gcc tcc gac acc gcc   1008
   Gly Leu Leu Glu Gln Ala Ala Ala Val Glu Glu Ala Ser Asp Thr Ala
                   325                 330                 335
   gcg gcg aac cag ttg atg aac aat gtg ccc cag gcg ctg caa cag ctg   1056
   Ala Ala Asn Gln Leu Met Asn Asn Val Pro Gln Ala Leu Gln Gln Leu
               340                 345                 350
   gcc cag ccc acg cag ggc acc acg cct tct tcc aag ctg ggt ggc ctg   1104
   Ala Gln Pro Thr Gln Gly Thr Thr Pro Ser Ser Lys Leu Gly Gly Leu
           355                 360                 365
   tgg aag acg gtc tcg ccg cat cgg tcg ccg atc agc aac atg gtg tcg   1152
   Trp Lys Thr Val Ser Pro His Arg Ser Pro Ile Ser Asn Met Val Ser
       370                 375                 380
   atg gcc aac aac cac atg tcg atg acc aac tcg ggt gtg tcg atg acc   1200
   Met Ala Asn Asn His Met Ser Met Thr Asn Ser Gly Val Ser Met Thr
   385                 390                 395                 400
   aac acc ttg agc tcg atg ttg aag ggc ttt gct ccg gcg gcg gcc gcc   1248
   Asn Thr Leu Ser Ser Met Leu Lys Gly Phe Ala Pro Ala Ala Ala Ala
                   405                 410                 415
   cag gcc gtg caa acc gcg gcg caa aac ggg gtc cgg gcg atg agc tcg   1296
   Gln Ala Val Gln Thr Ala Ala Gln Asn Gly Val Arg Ala Met Ser Ser
               420                 425                 430
   ctg ggc agc tcg ctg ggt tct tcg ggt ctg ggc ggt ggg gtg gcc gcc   1344
   Leu Gly Ser Ser Leu Gly Ser Ser Gly Leu Gly Gly Gly Val Ala Ala
           435                 440                 445
   aac ttg ggt cgg gcg gcc tcg gtc ggt tcg ttg tcg gtg ccg cag gcc   1392
   Asn Leu Gly Arg Ala Ala Ser Val Gly Ser Leu Ser Val Pro Gln Ala
       450                 455                 460
   tgg gcc gcg gcc aac cag gca gtc acc ccg gcg gcg cgg gcg ctg ccg   1440
   Trp Ala Ala Ala Asn Gln Ala Val Thr Pro Ala Ala Arg Ala Leu Pro
   465                 470                 475                 480
   ctg acc agc ctg acc agc gcc gcg gaa aga ggg ccc ggg cag atg ctg   1488
   Leu Thr Ser Leu Thr Ser Ala Ala Glu Arg Gly Pro Gly Gln Met Leu
                   485                 490                 495
   ggc ggg ctg ccg gtg ggg cag atg ggc gcc agg gcc ggt ggt ggg ctc   1536
   Gly Gly Leu Pro Val Gly Gln Met Gly Ala Arg Ala Gly Gly Gly Leu
               500                 505                 510
   agt ggt gtg ctg cgt gtt ccg ccg cga ccc tat gtg atg ccg cat tct   1584
   Ser Gly Val Leu Arg Val Pro Pro Arg Pro Tyr Val Met Pro His Ser
           515                 520                 525
   ccg gca gcc ggc gat atc gcc ccg ccg gcc ttg tcg cag gac cgg ttc    1632
   Pro Ala Ala Gly Asp Ile Ala Pro Pro Ala Leu Ser Gln Asp Arg Phe
       530                 535                 540
   gcc gac ttc ccc gcg ctg ccc ctc gac ccg tcc gcg atg gtc gcc caa    1680
   Ala Asp Phe Pro Ala Leu Pro Leu Asp Pro Ser Ala Met Val Ala Gln
   545                 550                 555                 560
   gtg ggg cca cag gtg gtc aac atc aac acc aaa ctg ggc tac aac aac    1728
   Val Gly Pro Gln Val Val Asn Ile Asn Thr Lys Leu Gly Tyr Asn Asn
                   565                 570                 575
   gcc gtg ggc gcc ggg acc ggc atc gtc atc gat ccc aac ggt gtc gtg    1776
   Ala Val Gly Ala Gly Thr Gly Ile Val Ile Asp Pro Asn Gly Val Val
               580                 585                 590
   ctg acc aac aac cac gtg atc gcg ggc gcc acc gac atc aat gcg ttc    1824
   Leu Thr Asn Asn His Val Ile Ala Gly Ala Thr Asp Ile Asn Ala Phe
           595                 600                 605
   agc gtc ggc tcc ggc caa acc tac ggc gtc gat gtg gtc ggg tat gac    1872
   Ser Val Gly Ser Gly Gln Thr Tyr Gly Val Asp Val Val Gly Tyr Asp
       610                 615                 620
   cgc acc cag gat gtc gcg gtg ctg cag ctg cgc ggt gcc ggt ggc ctg    1920
   Arg Thr Gln Asp Val Ala Val Leu Gln Leu Arg Gly Ala Gly Gly Leu
   625                 630                 635                 640
   ccg tcg gcg gcg atc ggt ggc ggc gtc gcg gtt ggt gag ccc gtc gtc    1968
   Pro Ser Ala Ala Ile Gly Gly Gly Val Ala Val Gly Glu Pro Val Val
                   645                 650                 655
   gcg atg ggc aac agc ggt ggg cag ggc gga acg ccc cgt gcg gtg cct    2016
   Ala Met Gly Asn Ser Gly Gly Gln Gly Gly Thr Pro Arg Ala Val Pro
               660                 665                 670
   ggc agg gtg gtc gcg ctc ggc caa acc gtg cag gcg tcg gat tcg ctg    2064
   Gly Arg Val Val Ala Leu Gly Gln Thr Val Gln Ala Ser Asp Ser Leu
           675                 680                 685
   acc ggt gcc gaa gag aca ttg aac ggg ttg atc cag ttc gat gcc gcg    2112
   Thr Gly Ala Glu Glu Thr Leu Asn Gly Leu Ile Gln Phe Asp Ala Ala
       690                 695                 700
   atc cag ccc ggt gat tcg ggc ggg ccc gtc gtc aac ggc cta gga cag    2160
   Ile Gln Pro Gly Asp Ser Gly Gly Pro Val Val Asn Gly Leu Gly Gln
   705                 710                 715                 720
   gtg gtc ggt atg aac acg gcc gcg tcc tag g                          2191
   Val Val Gly Met Asn Thr Ala Ala Ser
                   725                 730
<210> 12
   <211> 729
   <212> PRT
   <213> Artificial Sequence
   <223> Description of Artificial Sequence:Ra12-H9-32A fusion polypeptide (Ra12-MTB39-MTB32A(N-ter) fusion polypeptide)
<400> 12
   Met His His His His His His Thr Ala Ala Ser Asp Asn Phe Gln Leu
    1               5                  10                  15
   Ser Gln Gly Gly Gln Gly Phe Ala Ile Pro Ile Gly Gln Ala Met Ala
               20                  25                  30
   Ile Ala Gly Gln Ile Arg Ser Gly Gly Gly Ser Pro Thr Val His Ile
           35                  40                  45
   Gly Pro Thr Ala Phe Leu Gly Leu Gly Val Val Asp Asn Asn Gly Asn
       50                  55                  60
   Gly Ala Arg Val Gln Arg Val Val Gly Ser Ala Pro Ala Ala Ser Leu
   65                  70                  75                  80
   Gly Ile Ser Thr Gly Asp Val Ile Thr Ala Val Asp Gly Ala Pro Ile
                   85                  90                  95
   Asn Ser Ala Thr Ala Met Ala Asp Ala Leu Asn Gly His His Pro Gly
               100                 105                 110
   Asp Val Ile Ser Val Thr Trp Gln Thr Lys Ser Gly Gly Thr Arg Thr
           115                 120                 125
   Gly Asn Val Thr Leu Ala Glu Gly Pro Pro Ala Glu Phe Met Val Asp
       130                 135                 140
   Phe Gly Ala Leu Pro Pro Glu Ile Asn Ser Ala Arg Met Tyr Ala Gly
   145                 150                 155                 160
   Pro Gly Ser Ala Ser Leu Val Ala Ala Ala Gln Met Trp Asp Ser Val
                   165                 170                 175
   Ala Ser Asp Leu Phe Ser Ala Ala Ser Ala Phe Gln Ser Val Val Trp
               180                 185                 190
   Gly Leu Thr Val Gly Ser Trp Ile Gly Ser Ser Ala Gly Leu Met Val
           195                 200                 205
   Ala Ala Ala Ser Pro Tyr Val Ala Trp Met Ser Val Thr Ala Gly Gln
       210                 215                 220
   Ala Glu Leu Thr Ala Ala Gln Val Arg Val Ala Ala Ala Ala Tyr Glu
   225                 230                 235                 240
   Thr Ala Tyr Gly Leu Thr Val Pro Pro Pro Val Ile Ala Glu Asn Arg
                   245                 250                 255
   Ala Glu Leu Met Ile Leu Ile Ala Thr Asn Leu Leu Gly Gln Asn Thr
               260                 265                 270
   Pro Ala Ile Ala Val Asn Glu Ala Glu Tyr Gly Glu Met Trp Ala Gln
           275                 280                 285
   Asp Ala Ala Ala Met Phe Gly Tyr Ala Ala Ala Thr Ala Thr Ala Thr
       290                 295                 300
   Ala Thr Leu Leu Pro Phe Glu Glu Ala Pro Glu Met Thr Ser Ala Gly
   305                 310                 315                 320
   Gly Leu Leu Glu Gln Ala Ala Ala Val Glu Glu Ala Ser Asp Thr Ala
                   325                 330                 335
   Ala Ala Asn Gln Leu Met Asn Asn Val Pro Gln Ala Leu Gln Gln Leu
               340                 345                 350
   Ala Gln Pro Thr Gln Gly Thr Thr Pro Ser Ser Lys Leu Gly Gly Leu
           355                 360                 365
   Trp Lys Thr Val Ser Pro His Arg Ser Pro Ile Ser Asn Met Val Ser
       370                 375                 380
   Met Ala Asn Asn His Met Ser Met Thr Asn Ser Gly Val Ser Met Thr
   385                 390                 395                 400
   Asn Thr Leu Ser Ser Met Leu Lys Gly Phe Ala Pro Ala Ala Ala Ala
                   405                 410                 415
   Gln Ala Val Gln Thr Ala Ala Gln Asn Gly Val Arg Ala Met Ser Ser
               420                 425                 430
   Leu Gly Ser Ser Leu Gly Ser Ser Gly Leu Gly Gly Gly Val Ala Ala
           435                 440                 445
   Asn Leu Gly Arg Ala Ala Ser Val Gly Ser Leu Ser Val Pro Gln Ala
       450                 455                 460
   Trp Ala Ala Ala Asn Gln Ala Val Thr Pro Ala Ala Arg Ala Leu Pro
   465                 470                 475                 480
   Leu Thr Ser Leu Thr Ser Ala Ala Glu Arg Gly Pro Gly Gln Met Leu
                   485                 490                 495
   Gly Gly Leu Pro Val Gly Gln Met Gly Ala Arg Ala Gly Gly Gly Leu
               500                 505                 510
   Ser Gly Val Leu Arg Val Pro Pro Arg Pro Tyr Val Met Pro His Ser
           515                 520                 525
   Pro Ala Ala Gly Asp Ile Ala Pro Pro Ala Leu Ser Gln Asp Arg Phe
       530                 535                 540
   Ala Asp Phe Pro Ala Leu Pro Leu Asp Pro Ser Ala Met Val Ala Gln
   545                 550                 555                 560
   Val Gly Pro Gln Val Val Asn Ile Asn Thr Lys Leu Gly Tyr Asn Asn
                   565                 570                 575
   Ala Val Gly Ala Gly Thr Gly Ile Val Ile Asp Pro Asn Gly Val Val
               580                 585                 590
   Leu Thr Asn Asn His Val Ile Ala Gly Ala Thr Asp Ile Asn Ala Phe
           595                 600                 605
   Ser Val Gly Ser Gly Gln Thr Tyr Gly Val Asp Val Val Gly Tyr Asp
       610                 615                 620
   Arg Thr Gln Asp Val Ala Val Leu Gln Leu Arg Gly Ala Gly Gly Leu
   625                 630                 635                 640
   Pro Ser Ala Ala Ile Gly Gly Gly Val Ala Val Gly Glu Pro Val Val
                   645                 650                 655
   Ala Met Gly Asn Ser Gly Gly Gln Gly Gly Thr Pro Arg Ala Val Pro
               660                 665                 670
   Gly Arg Val Val Ala Leu Gly Gln Thr Val Gln Ala Ser Asp Ser Leu
           675                 680                 685
   Thr Gly Ala Glu Glu Thr Leu Asn Gly Leu Ile Gln Phe Asp Ala Ala
       690                 695                 700
   Ile Gln Pro Gly Asp Ser Gly Gly Pro Val Val Asn Gly Leu Gly Gln
   705                 710                 715                 720
   Val Val Gly Met Asn Thr Ala Ala Ser
                   725
<210> 13
   <211> 51
   <212> DNA
   <213> Artificial Sequence
<220>
   <223> Description of Artificial Sequence:oligonucleotide primer for PCR amplification of Ra12 C-terminal fragment of MTB32A
<400> 13
   caattacata tgcatcacca tcaccatcac acggccgcgt ccgataactt c 51
<210> 14
   <211> 33
   <212> DNA
   <213> Artificial Sequence
<220>
   <223> Description of Artificial Sequence:3' oligonucleotide primer for PCR amplification of Ra12 C-terminal fragment of MTB32A
<400> 14
   ctaatcgaat tcggccgggg gtccctcggc caa 33
<210> 15
   <211> 48
   <212> DNA
   <213> Artificial Sequence
<220>
   <223> Description of Artificial Sequence:5' oligonucleotide primer containing enterokinase recognition site for PCR amplification of DPPD mature secreted form
<400> 15
   caattagaat tcgacgacga cgacaaggat ccacctgacc cgcatcag 48
<210> 16
   <211> 33
   <212> DNA
   <213> Artificial Sequence
<220>
   <223> Description of Artificial Sequence:3' oligonucleotide primer containing enterokinase recognition site for PCR amplification of DPPD mature secreted form
<400> 16
   caattagaat tctcagggag cgttgggctg ctc 33
<210> 17
   <211> 30
   <212> PRT
   <213> Artificial Sequence
<220>
   <223> Description of Artificial Sequence:Ra12(short) polypeptide
<400> 17
   Thr Ala Ala Ser Asp Asn Phe Gln Leu Ser Gln Gly Gly Gln Gly Phe
     1               5                  10                  15
   Ala Ile Pro Ile Gly Gln Ala Met Ala Ile Ala Gly Gln Ile
                20                  25                  30
<210> 18
   <211> 128
   <212> PRT
   <213> Artificial Sequence
<220>
   <223> Description of Artificial Sequence:Ra12(long) polypeptide
<400> 18
   Thr Ala Ala Ser Asp Asn Phe Gln Leu Ser Gln Gly Gly Gln Gly Phe
     1               5                  10                  15
   Ala Ile Pro Ile Gly Gln Ala Met Ala Ile Ala Gly Gln Ile Lys Leu
                20                  25                  30
   Pro Thr Val His Ile Gly Pro Thr Ala Phe Leu Gly Leu Gly Val Val
               35                  40                  45
   Asp Asn Asn Gly Asn Gly Ala Arg Val Gln Arg Val Val Gly Ser Ala
           50                  55                  60
   Pro Ala Ala Ser Leu Gly Ile Ser Thr Gly Asp Val Ile Thr Ala Val
   65                  70                  75                  80
   Asp Gly Ala Pro Ile Asn Ser Ala Thr Ala Met Ala Asp Ala Leu Asn
                   85                  90                  95
   Gly His His Pro Gly Asp Val Ile Ser Val Thr Trp Gln Thr Lys Ser
               100                 105                 110
   Gly Gly Thr Arg Thr Gly Asn Val Thr Leu Ala Glu Gly Pro Pro Ala
           115                 120                 125
<210> 19
   <211> 34
   <212> DNA
   <213> Artificial Sequence
<220>
   <223> Description of Artificial Sequence:5' oligonucleotide primer, HindIII site, for PCR amplification of human mammaglobin
<400> 19
   gcgaagctta tgaagttgct gatggtcctc atgc 34
<210> 20
   <211> 36
   <212> DNA
   <213> Artificial Sequence
<220>
   <223> Description of Artificial Sequence:3' oligonucleotide primer, XhoI site, for PCR amplification of human mammaglobin
<400> 20
   cggctcgagt taaaataaat cacaaagact gctgtc 36
<210> 21
   <211> 7
   <212> PRT
   <213> Artificial Sequence
<220>
   <223> Description of Artificial Sequence:Met-His tag 6aa
<400> 21
   Met His His His His His His
   1               5
<210> 22
   <211> 4
   <212> PRT
   <213> Artificial Sequence
<220>
   <223> Description of Artificial Sequence:enterokinase recognition site
<400> 22
   Asp Asp Asp Lys
    1